(19) Europäisches Patentamt
European Patent Office
Office européen des brevets

(11) EP 1 660 475 B1

(12) EUROPEAN PATENT SPECIFICATION

(45) Date of publication and mention of the grant of the patent:
**28.05.2008 Bulletin 2008/22**

(21) Application number: **04763883.8**

(22) Date of filing: **06.08.2004**

(51) Int Cl.:
***C07D 401/04*** *(2006.01)* ***C07D 519/00*** *(2006.01)*
***A61K 31/506*** *(2006.01)* ***A61K 31/52*** *(2006.01)*
***A61P 35/00*** *(2006.01)* ***A61P 25/28*** *(2006.01)*
***A61P 31/12*** *(2006.01)* ***A61P 37/00*** *(2006.01)*
***A61P 13/08*** *(2006.01)* ***A61P 17/06*** *(2006.01)*
***A61P 9/10*** *(2006.01)* ***A61P 11/00*** *(2006.01)*
***A61P 19/02*** *(2006.01)* ***A61P 13/12*** *(2006.01)*
***A61K 45/06*** *(2006.01)*

(86) International application number:
**PCT/EP2004/008853**

(87) International publication number:
**WO 2005/014572 (17.02.2005 Gazette 2005/07)**

# (54) PYRIMIDYLPYRROLE DERIVATIVES ACTIVE AS KINASE INHIBITORS

PYRIMIDYLPYRROLDERIVATE, DIE ALS KINASEINHIBITOREN WIRKEN

DERIVES DE PYRIMIDYLPYRROLE POUVANT ETRE UTILISES COMME INHIBITEURS DE KINASES

(84) Designated Contracting States:
**AT BE BG CH CY CZ DE DK EE ES FI FR GB GR HU IE IT LI LU MC NL PL PT RO SE SI SK TR**

(30) Priority: **08.08.2003 US 493633 P**

(43) Date of publication of application:
**31.05.2006 Bulletin 2006/22**

(73) Proprietor: **Pfizer Italia S.r.l.**
**04010 Latina (IT)**

(72) Inventors:
- **VANOTTI, Ermes**
  **I-20148 Milano (IT)**
- **D'ALESSIO, Roberto**
  **I-20092 Cinisello Balsamo (Milan) (IT)**
- **TIBOLLA, Marcellino**
  **I-20030 Senago (Milano) (IT)**
- **VARASI, Mario**
  **I-20142 Milano (IT)**
- **MONTAGNOLI, Alessia**
  **I-20151 Milan (IT)**
- **SANTOCANALE, Corrado**
  **I-22070 Appiano Gentile, Como (IT)**
- **ORSINI, Paolo**
  **I-20025 Legano, Milan (IT)**
- **PILLAN, Antonio**
  **I-20133 Milan (IT)**

(74) Representative: **Modiano, Micaela Nadia et al**
**Modiano Josif Pisanty & Staub Ltd**
**Thierschstrasse 11**
**80538 München (DE)**

(56) References cited:
**WO-A-02/12242** **WO-A-03/027114**

- **DATABASE HCAPLUS [Online] CHEMICAL ABSTRACTS SERVICE, COLUMBUS, OHIO, US; ANDERSON, DAVID R. ET AL: "Preparation of pyrrolopyridinones as mitogen activated protein kinase-activated protein kinase-2 inhibiting compounds" XP002305840 retrieved from STN Database accession no. 2004:633665 -& WO 2004/058762 A (MAHONEY MATTHEW W ; PHILLION DENNIS P (US); VERNIER WILLIAM F (US); PO) 15 July 2004 (2004-07-15) cited in the application**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### Field of the Invention

The present invention relates to pyrimidylpyrrole derivatives, to a process for their preparation, to pharmaceutical compositions comprising them, and to their use as therapeutic agents, particularly in the treatment of cancer and cell proliferation disorders.

### Discussion of the Background

The malfunctioning of protein kinases (PKs) is the hallmark of numerous diseases. A large share of the oncogenes and proto-oncogenes involved in human cancers code for PKs. The enhanced activities of PKs are also implicated in many non-malignant diseases, such as benign prostate hyperplasia, familial adenomatosis, polyposis, neuro-fibromatosis, psoriasis, vascular smooth cell proliferation associated with atherosclerosis, pulmonary fibrosis, arthritis glomerulonephritis and post-surgical stenosis and restenosis.
PKs are also implicated in Inflammatory conditions and in the multiplication of viruses and parasites. PKs may also play a major role in the pathogenesis and development of neurodegenerative disorders.
For a general reference to PKs malfunctioning or disregulation see, for instance, Current Opinion in Chemical Biology 1999, 3, 459 - 465.
Among the several protein kinases known in the art as being Implicated in the growth of cancer cells is Cdc7, an evolutionary conserved serine-threonine kinase which plays a pivotal role in linking cell cycle regulation to genome duplication, being essential for the firing of DNA replication origins (see Montagnoli A. et al., EMBO Journal, Vol. 21, No. 12, pp. 3171-3181, 2002).
WO 03/027114 discloses 1, 5, 6, 7-tetrahydropyrrolo[3,2-c]pyridine derivatives which have been found to suppress appetite and induce weight loss.

### SUMMARY OF THE INVENTION

It is an object of the invention to provide compounds which are useful, in therapy, as agents against a host of diseases caused by and/or associated to a disregulated protein kinase activity and, more particularly, Cdk2 and Cdc7 activity.
It Is another object to provide compounds, which are endowed with protein kinase inhibiting activity and, more particularly, Cdk2 and Cdc7 inhibiting activity.
The present inventors have now discovered that some pyridylpyrroles are endowed with protein kinase inhibiting activity, e.g. Cdk2 and especially Cdc7 inhibiting activity.
More specifically, the compounds of this invention are useful in the treatment of a variety of cancers including: carcinoma such as bladder, breast, colon, kidney, liver, lung, including small cell lung cancer, esophagus, gall-bladder, ovary, pancreas, stomach, cervix, thyroid, prostate, and skin, Including squamous cell carcinoma; hematopoietic tumors of lymphoid lineage, including leukemia, acute lymphocitic leukemia, acute lymphoblastic leukemia, B-cell lymphoma, T-cell-lymphoma, Hodgkin's lymphoma, non-Hodgkin's lymphoma, hairy cell lymphoma and Burkett's lymphoma; hematopoietic tumors of myeloid lineage, Including acute and chronic myelogenous leukemias, myelodysplastic syndrome and promyelocytic leukemia; tumors of mesenchymal origin, including fibrosarcoma and rhabdomyosarcoma; tumors of the central and peripheral nervous system, Including astrocytoma, neuroblastoma, glioma and schwannomas; other tumors, including melanoma, seminoma, teratocarcinoma, osteosarcoma, xeroderma pigmentosum, keratoxanthoma, thyroid follicular cancer and Kaposi's sarcoma.
Due to the key role of PKs, Cdk2 and Cdc7 In the regulation of cellular proliferation, these pyrimidylpyrroles are also useful in the treatment of a variety of cell proliferative disorders such as benign prostate hyperplasia, familial adenomatosis, polyposis, neuro-fibromatosis, psoriasis, vascular smooth cell proliferation associated with atherosclerosis, pulmonary fibrosis, arthritis, glomerulonephritis and post-surgical stenosis and restenosis.
The compounds of the invention can be also active as inhibitors of other protein kinases such as, for instance, protein kinase C in different isoforms, Met, PAK-4, PAK-5, ZC-1, STLK-2, DDR-2, Aurora 1, Aurora 2, Bub-1, PLK, Chk1, Chk2, HER2, raf1, MEK1, MAPK, EGF-R, PDGF-R, FGF-R, IGF-R, VEGF-R, PI3K, weel kinase, Src, Abl, Akt, ILK, MK-2, IKK-2, Cdk In different isoforms, Nek, and thus be effective in the treatment of diseases associated with other protein kinases.
Accordingly, in a first embodiment, the present invention provides the use of a compound of formula (I) for the manufacture of a medicament for treating cell proliferative disorders caused by and/or associated with an altered protein kinase activity selected from the group consisting of cancer, Alzheimer's disease, viral infections, auto-immune diseases, neurodegenerative disorders, benign prostate hyperplasia, familial adenomatosis, polyposis, neuro-fibromatosis, psoriasis, vascular smooth cell proliferation associated with atherosclerosis, pulmonary fibrosis, arthritis, glomerulonephritis and post-surgical stenosis and restenosis, wherein the compound is to be administered in an effective amount to a mammal in need thereof,

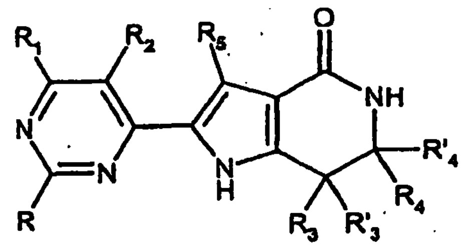

(I)

wherein
R is a hydrogen atom or a group selected from amino, arylamino, optionally substituted $C_1$-$C_6$ alkylamino, optionally substituted arylalkylamino, heteroarylalkylamino, $C_1$-$C_6$ dialkylamino and acylamino;
$R_1$ and $R_2$ are, each independently, a hydrogen or halogen atom, a straight or branched $C_1$-$C_6$ alkyl group, an amino or arylamino group or, taken together with the pyrimidine bond to which they are linked, $R_1$ and $R_2$ may form a divalent -NH-CH=N-, -N=CH-NH- or -NH-CH=CH- group;
$R_3$, $R'_3$, $R_4$ and $R'_4$ are, each independently, a hydrogen atom or a group selected from straight or branched $C_1$-$C_6$ alkyl, $C_3$-$C_6$ cycloalkyl, heterocyclyl, aryl, cycloalkyl-alkyl, heterocyclyl-alkyl or aryl-alkyl; or $R_3$ and $R_3$' or $R_4$ and $R_4$', taken together, form a $C_3$-$C_6$ cyclic alkyl group;
$R_5$ is a hydrogen or halogen atom or a straight or branched $C_1$-$C_6$ alkyl group and pharmaceutically acceptable salts thereof.

The above use enables treatment of cell proliferative disorders caused by and/or associated with an altered Cdc7 kinase activity.

In a preferred embodiment of the use described above, the cell proliferative disorder is cancer.

Specific types of cancer that may be treated Include carcinoma, squamous cell carcinoma, hematopoietic tumors of myeloid or lymphoid lineage, tumors of mesenchymal origin, tumors of the central and peripheral nervous system, melanoma, seminoma, teratocarcinoma, osteosarcoma, xeroderma pigmentosum, keratoxanthoma, thyroid follicular cancer, and Kaposl's sarcoma.

The present invention also provides a pyrimidylpyrrole derivative which is represented by formula (I)

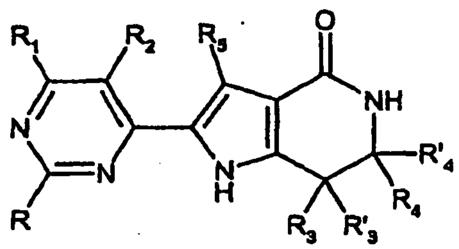

(I)

wherein
R is a hydrogen atom or a group selected from amino, arylamino, optionally substituted $C_1$-$C_6$ alkylamino, optionally substituted arylalkylamino, heteroarylalkylamino, $C_1$-$C_6$ dialkylamino and acylamino;
$R_1$ and $R_2$ are, each Independently, a hydrogen or halogen atom, a straight or branched $C_1$-$C_6$ alkyl group, an amino or arylamino group or, taken together with the pyrimidine bond to which they are linked, $R_1$ and $R_2$ may form a divalent -NH-CH=N-. -N=CH-NH- or -NH-CH=CH-group;
$R_3$, $R'_3$, $R_4$ and $R'_4$ are, each independently, a hydrogen atom or a group selected from straight or branched $C_1$-$C_6$ alkyl, $C_3$-$C_6$ cycloalkyl, heterocyclyl, aryl, cyctoalkyl-alkyl, heterocyclyl-alkyl or aryl--alkyl; or $R_3$ and $R_3$' or $R_4$ and $R_4$', taken together, form a $C_3$-$C_8$ cyclic alkyl group;
$R_5$ is a hydrogen or halogen atom or a straight or branched $C_1$-$C_6$ alkyl group and pharmaceutically acceptable salts thereof, provided that the compound 2-[2-(dimethylamino)-5-fluoropyrimidin-4-yl]-1,5,6,7-tetrahydro-4H-pyrrolo[3,2-C] pyridin-4-one is excluded.

The present invention also includes methods of synthesizing the pyrimidylpyrrole derivatives of formula (I), and

the pharmaceutical compositions comprising them.

A more complete appreciation of the invention as claimed in appended claims 1-18 and many of the attendant advantages thereof will be readily obtained as the same becomes better understood by reference to the following detailed description.

## DETAILED DESCRIPTION OF THE INVENTION

Several heterocyclic compounds are known In the art as protein kinase Inhibitors. Among them are, for instance, pyrrolo-pyrazoles disclosed in WO 02/12242; tetrahydroindazoles disclosed in WO 00/69846; pyrrolo-pyridines disclosed in WO 01/98299; aminophthalazinones disclosed in WO 03/014090 and aminoindazoles disclosed in WO. 03/028720.

In addition, pyridylpyrrole derivatives endowed with mitogen activated protein kinase-activated protein kinase-2 inhibitory activity are disclosed in the published PCT International Patent Application WO 04/058762 (published July 15, 2004), claiming priority of U.S. Serial No. 60/434,962, filed in December 12, 2002.

Among the compounds therein disclosed are, in particular, pyridylpyrroles which are substituted by aryl- or aryl-alkenyl- groups at the pyridine moiety; pyridylpyrroles being substituted by amino groups or halogen atoms at this same pyridine ring are also therein disclosed as synthetic intermediates.

Interestingly, the compounds of the invention fall within the broad general formula disclosed In the aforementioned patent application US 60/434962 but are not specifically exemplified therein.

The compounds of formula (I) of the invention may have asymmetric carbon atoms and may therefore exist as individual optical Isomers, as racemic admixtures or as any other admixture including a majority of one of the two optical isomers, which are all to be intended as comprised within the scope of the present invention.

Likewise, the use as an antitumor agent of all the possible isomers and their admixtures of the compounds of formula (I) are also within the scope of the present invention.

In cases when compounds may exist in tautomeric forms, for instance keto-enol tautomers, each tautomeric form is contemplated as being included within this invention whether existing in equilibrium or predominantly in one form.

In the present description, unless otherwise specified, with the term straight or branched $C_1$-$C_6$ alkyl we intend any of the groups such as methyl, ethyl, n-propyl, isopropyl, n-butyl. Isobutyl, tert-butyl, see-butyl, n-pentyl, n-hexyl.

With the term amino we intend an $-NH_2$ group whilst the term arylamino comprises any group -NH-aryl, wherein aryl is as defined below.

With the term aryl we Intend any carbocyclic or heterocyclic hydrocarbon with from 1 to 2 ring moieties, either fused or linked to each other by single bonds, wherein at least one of the rings is aromatic. If present, any aromatic heterocyclic hydrocarbon also referred to as heteroaryl group, comprises a 5 to 6 membered ring with from 1 to 3 heteroatoms selected among N, O or S.

Examples of aryl groups according to the invention are, for instance, phenyl, biphenyl, $\alpha$- or $\beta$-naphthyl, dihydronaphthyl, thienyl, benzothienyl, furyl, benzofuranyl, pyrrolyl, Imidazolyl, pyrazolyl, thiazolyl, Isothiazolyl, oxazolyl, isoxazolyl, pyridyl, pyrazinyl, pyrimidinyl, pyridazinyl, indolyl, isoindolyl, purinyl, quinolyl, isoquinolyl, dihydroquinolinyl, quinoxalinyl, benzodioxolyl, indanyl, indanyl, triazolyl.

With the term $C_3$-$C_6$ cycloalkyl we intend any group such as cyclopropyl, cyclobutyl, cyclopentyl and cyclohexyl.

With the term heterocyclyl we intend any 5 or 6 membered heterocyclic ring comprising from 1 to 3 heteroatoms selected among N, O or S. Clearly, if the said heterocycle or heterocyclyl group is an aromatic heterocycle, also referred to as heteroaryl, it is encompassed by the above definition given to aryl groups.

As such, besides the above aromatic heterocycles, the term heterocyclyl also encompasses saturated or partially unsaturated heterocycles such as, for instance, pyrroline, pyrrolidine, imidazoline, imidazolidine, pyrazoline, pyrazolidine, piperidine, piperazine, morpholine.

With the term acylamino we intend any of $C_1$-$C_6$ alkylcarbonylamino, $C_3$-$C_7$ cycloalkylcarbonylamino, cycloalkylalkylcarbonylamino group, any arylcarbonylamino, heteroarylcarbonylamino, arylalkylcarbonylamino and heteroarylalkylcarbonylamino group. From the above, it is clear to the skilled person that any group whose name is identified as a composite name such as, for instance, cycloalkyl-alkyl, heterocyclyl-alkyl, arylalkyl and the like, have all to be intended as construed by the moieties from which they derive. In this respect, as an example, any group which is identified as an arylalkyl has to be intended as an alkyl group which is further substituted by aryl, wherein both aryl and alkyl are as above defined.

Clearly when $R_3$ and $R_3$ or $R_4$ and $R_4$', taken together, form a $C_3$-$C_6$ cyclic alkyl group, the compound is referred to as spiro derivative.

When alkylamino or arylalkylamino group is optionally substituted, the substituents are chosen from alkyl, cycloalkyl, haloalkyl, amino, hydroxy, alkoxy, halogen, alkoxycarbonyl, aminocarbonyl and alkylcarbonylamino as herein defined.

Clearly, when $R_1$ and $R_2$ are linked together as forming any divalent group -NH-CH=N-, -N=CH-NH- or -NH-CH=CH-, purine and fused pyrimidine-pyrrole systems are obtained as having the following formulae, still being an object

of the invention.

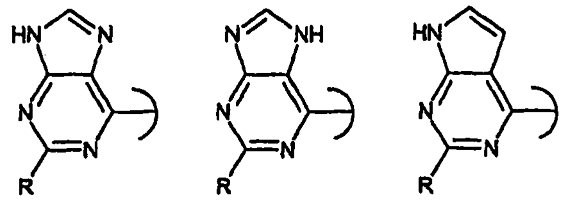

Pharmaceutically acceptable salts of the compounds of formula (I) include the acid addition salts with inorganic or organic acids such as, for instance, nitric, hydrochloric, hydrobromic, sulfuric, perchloric, phosphoric, acetic, trifluoroacetic, propionic, glycolic, lactic, oxalic, malonic, malic, maleic, tartaric, citric, benzoic, cinnamic, mandelic, methanesulphonic, isethionic and salicylic acid.

A preferred class of compounds of the invention is represented by the derivatives of formula (I) wherein R is hydrogen, amino or phenylamino; $R_1$ and $R_2$ are both hydrogen atoms or, taken together with the pyrimidine bond to which they are linked, form a divalent -NH-CH=N-group; and $R_3$, $R'_3$, $R_4$, $R'_4$ and $R_5$ are as above defined.

Another class of preferred compounds of the invention is represented by the derivatives of formula (I) wherein at least one of $R_3$ and $R'_3$ is a hydrogen atom.

Another class of preferred compounds of the invention is represented by the derivatives of formula (I) wherein at least one of $R_4$ and $R'_4$ is a hydrogen atom.

Still more preferred compounds of the invention are the derivatives of formula (I) wherein $R_1$, $R_2$ are both hydrogen atoms.

For a reference to any specific compound of formula (i) of the invention, optionally in the form of a pharmaceutically acceptable salt, see the experimental section and claims.

As formerly indicated, a further object of the present invention is represented by the process for preparing the compounds of formula (I).

The compounds of formula (I) may be prepared according to the following synthetic scheme, by reacting the pyrimidine derivative of formula (III) with a suitable piperidine-dione derivative of formula (IV) wherein Q is H or a suitable nitrogen protecting group, preferably tert-butoxycarbonyl.

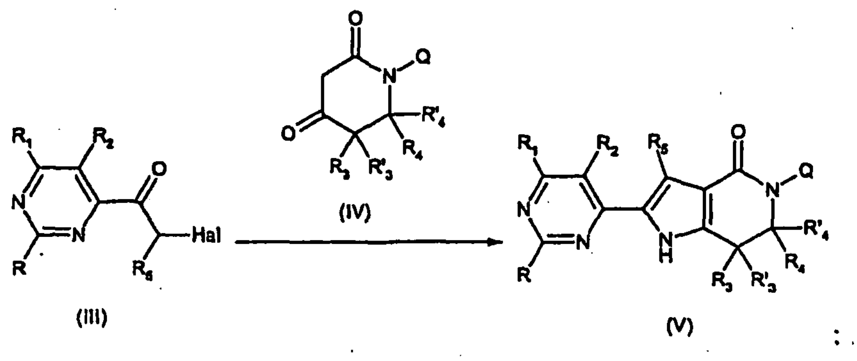

The reaction occurs in the presence of ammonium acetate and of a suitable solvent such as, for instance, a lower alcohol. Preferably, the reaction is carried out in the presence of ethanol by working at room temperature and for a suitable time varying from about 2 hours to about 24 hours. If necessary compound (V) is converted into compound (I) by removal of the protecting group Q.

Therefore, the compounds of formula (I) and the pharmaceutically acceptable salts thereof may be obtained by a process comprising:

a) halogenating a compound of formula (II) so as to obtain a compound of formula (III)

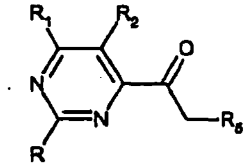

(II)

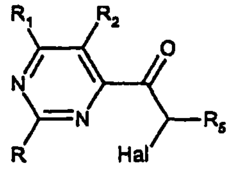

(III)

wherein R, $R_1$ and $R_2$ have the above reported meanings, $R_5$ is a hydrogen atom or a straight or branched $C_1$-$C_6$ alkyl group and Hal represents a suitable halogen atom, preferably bromine or chlorine;
b) reacting the compound of formula (III) with a compound of formula (N)

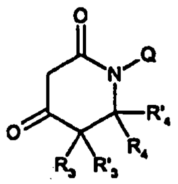

(IV)

wherein $R_3$, $R'_3$, $R_4$, $R'_4$ and Q have the above reported meanings, so as to obtain a compound of formula (I) and, optionally, converting it into another compound of formula (I) and/or into a pharmaceutically acceptable salt thereof.

The above process is an analogy process which can be carried out according to methods well known in the art.

According to step (a) of the process the compound of formula (II) is halogenated, in particular brominated or chlorinated, by working under conventional methods, for instance in the presence of bromine and in a suitable solvent such as acetic and hydrobromic acid mixtures, for a time varying from 1 hour to 24 hours. Alternatively, a suitable activated derivative of the compound of formula (11), for instance an enolether or silylether, can be reacted with a halogen source such as N-bromo-succinimide (NBS) in a suitable solvent such as, for instance, tetrahydrofuran/water mixtures.

According to step (b) of the process, the intermediate compound of formula (III) is then reacted with a compound of formula (IV), in the presence of ammonium acetate and of a suitable solvent such as, for instance, a lower alcohol. Preferably, the reaction is carried out in the presence of ethanol by working at room temperature and for a suitable time varying from 2 hours to 24 hours.

The final compound of formula (I) thus obtained may be then converted into another compound of formula (I) according to well-known methods in the art. As an example, the compounds of formula (I) wherein $R_5$ represents a hydrogen atom can be easily converted into the corresponding compounds of formula (I) wherein $R_5$ is a halogen atom, through conventional methods reported in the literature for the halogenation of pyrrole derivatives.

In another example, the compounds of formula (I), wherein R is an opt. substituted $C_1$-$C_6$ alkylamino or opt. substituted arylalkylamino or heteroarylalkylamino or $C_1$-$C_6$ dialkylamino can be obtained from another compound of formula (I) where R is amino by reductive amination.

Another example is represented by the preparation of compounds of formula (I), wherein R is an acylamino group by reacting a compound of formula (V), that is a protected form of a compound of formula (I), with a suitable acyl halide, as shown in the following scheme:

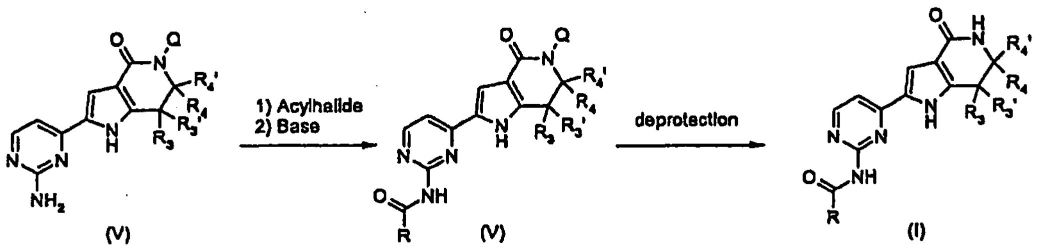


(V) (V) (I)

Likewise, the conversion of a compound of formula (I) into a pharmaceutically acceptable salt is easily carried

out according to known methods, e.g. by contacting any free base of formula (I) with any suitable pharmaceutically acceptable acid.

The compounds of formula (II) and (IV), as well as any other reactant of the process, are known or they can be easily prepared according to known methods.

As an example, the compounds of formula (II) wherein R is amino ($-NH_2$) or arylamino (-NH-Ar) may be obtained according to the following path:

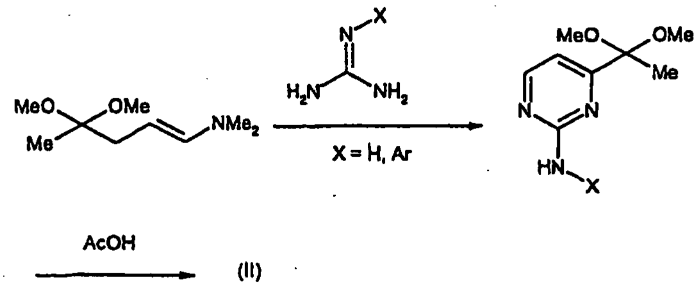

(II)

1-(Dimethylamino)-4,4-dimethoxy-1-penten-3-one is a known compound which can be prepared according to known methods, for instance as reported In J. Het. Chem., 22(6), 1723-6, 1985. It is easily reacted with guanidine or a guanidine derivative thereof, for instance being available in the form of an acid addition salt, e.g. as guanidinium hydrochloride salt. The reaction is carried out under basic conditions, for instance in the presence of sodium ethylate and of a suitable solvent such as a lower alcohol, preferably ethanol. The reaction occurs at refluxing temperature, for a suitable time up to 24 hours.

In their turn, guanidine and derivatives thereof are known and if not commercially available per se can be easily prepared according to known methods.

The above reaction allows to obtain the intermediate pyrimidine compound which is then converted into the compound of formula (11) through acidic treatment at room temperature, for instance In the presence of acetic acid.

Likewise, the compounds of formula (11) wherein $R_1$ and $R_2$ are linked together through the divalent -N=CH-NH- group so as to form a purine system, may be obtained from commercially available 6-chloro-9H-purine. In this respect, the halogenated derivative of formula (III) may be directly obtained as per the synthetic scheme below, without the need of isolating the intermediate purine compound of formula (II):

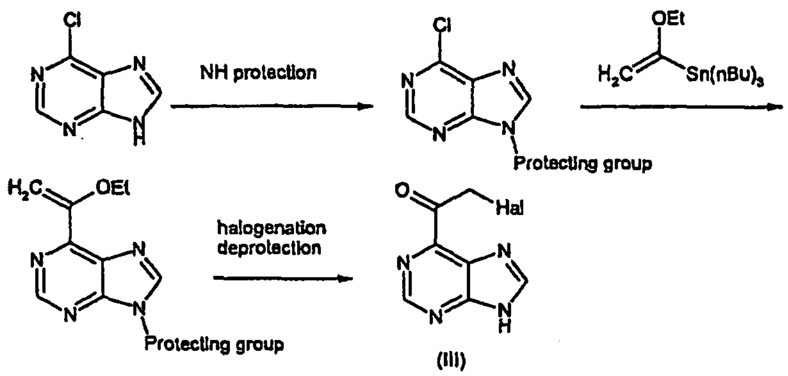

(III)

6-Chloro-gH-purine is thus protected at the imidazole nitrogen atom according to conventional methods, for instance with 3,4-dihydro-2H-pyran in the presence of p-toluenesulfonic acid, so as to get the corresponding 6-chloropurine derivative wherein the protecting group is, e.g., tetrahydro-2H-pyran-2-yl.

This derivative is then reacted with 1-(ethoxyvinyl)tributyltin and in the presence of palladium tetrakis triphenylphosphine so as to obtain the corresponding 6-(1-ethoxyvinyl)purine derivative. This latter intermediate compound is then directly converted into the halogenated derivative of formula (III) with proper halogenating agents and in the presence of suitable solvents, as formerly reported.

By working in an analogous method and by using any proper starting material, additional compounds of formula (11), for instance those wherein $R_1$ and $R_2$ form a divalent -NH-CH=CH- group, may be thus obtained.

The compounds of formula (I) wherein one or both of $R_1$ and $R_2$ are other than hydrogen atoms are prepared

according to the process object of the invention by starting from the corresponding pyrimidine derivatives of formula (II).

Also the piperidine-dione derivative (IV) is a known compound or, alternatively, can be prepared by known methods, for instance according to the synthetic pathway below, wherein Alk stands for a suitable lower alkyl group, e.g. ethyl, and A stands for chloro or OAlk:

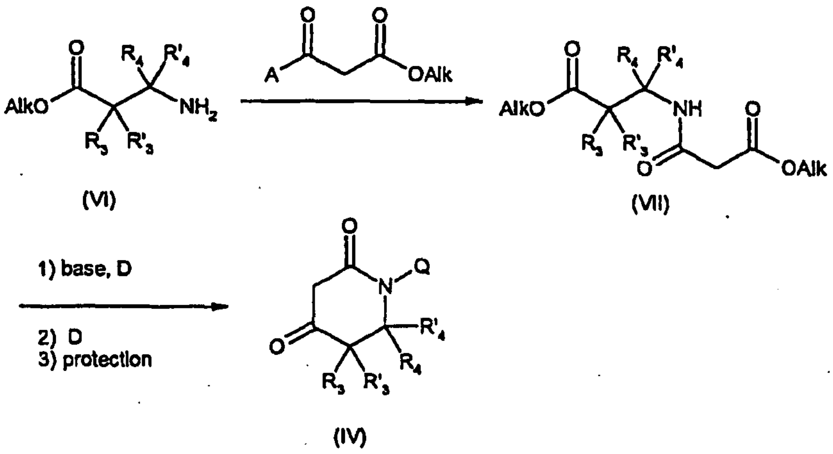


(VI) (VII) (IV)

In this respect, a suitable β-anilno-carboxyester (VI) derivative wherein $R_3$, $R'_3$, $R_4$ and $R'_4$ have the above reported meanings, is reacted with dialkylmalonate or, alternatively, with 3-chioro-3-oxopropanoic acid alkyl ester, for instance, dimethylmalonate or ethyl 3-chloro-3-oxopropanoate, respectively. When A is chloro the reaction is carried out under basic conditions, for instance in the presence of triethylamine, and in a suitable solvent such as dichloromethane, at a temperature comprised between room temperature to reflux. When A is OAlk the reaction is carried out with or without basic conditions and more conveniently in the absence of solvents at reflux temperature of the dialkylmalonate.

When not commercially available, the above mentioned β-amino-carboxyester derivatives (VI) can be obtained according to well known procedures described in the literature.

The intermediate derivative thus obtained (VII) is then converted into the compound of formula (IV) In a two-steps reaction, by first reacting it under basic conditions, e.g. in the presence of sodium methylate and of a suitable solvent, preferably toluene, at refluxing temperature and for a time varying between 2 hours and 24 hours. Subsequently, the product of the former step is reacted as such, without being isolated, with an acetonitrile/water/acetic acid mixture under refluxing conditions and for a time varying between 12 hours and 24 hours. Optionally the piperidin-dione (IV) can be protected with a suitable protecting group Q.

In the alternative, the piperidine-dione derivative (IV) can be prepared, for instance, according also to the alternative synthetic pathway below:

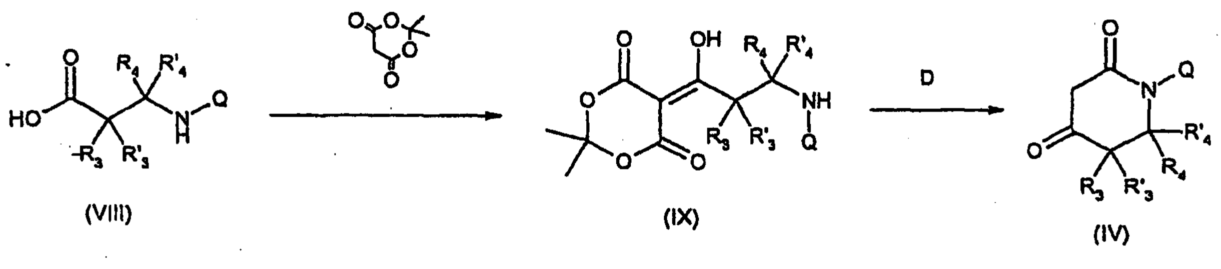


(VIII) (IX) (IV)

In the procedure, Meldrum's acid (2,2-dimethyl-1,3-dioxane-4,6-dione) is reacted with a suitable aminoacid derivative of formula (VIII) so as to obtain a compound of formula (IX) wherein Q is a suitable nitrogen protecting group and $R_3$, $R'_3$, $R_4$ and $R'_4$ are as above defined. The compound of formula (IX) is then cyclized by dissolving it in a suitable solvent, for instance ethylacetate, and refluxing for a period of time from 1 to 24 hours;

or, in the alternative, the piperidine-dione derivative (IV) can be modified according to the synthetic pathway below, wherein Q stands for a suitable nitrogen-protecting group such as, In particular, tert-butoxycarbonyl, or other groups, such as p-methoxybenzyl, p-methoxyethylbenzyl, p-methoxyphenyl:

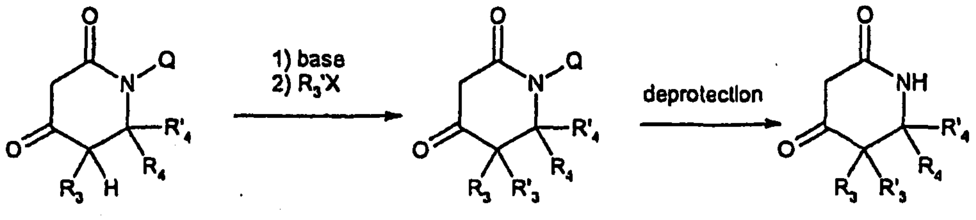


(IV) (IV) (IV)

In this respect, a suitable piperidine-dione derivative (IV) wherein $R_3$, $R_4$ and $R'_4$ and Q have the above reported meanings, is reacted with a base, for instance lithium bis(trimethylsilyl)amide (LIHMDS). The reaction is carried out in a suitable solvent such as tetrahydrofuran, at a temperature comprised between -78°C and room temperature.
The reaction mixture is then treated with a suitable alkyl halide thus obtaining another compound of formula, (IV). The compound thus obtained, where Q is for instance a tert-butoxycarbonyl group, can be converted into another compound of formula (IV) by treating it with acidic conditions, e.g. in the presence of trifluoroacetic acid and of a suitable solvent, preferably dichloromethane, at room temperature and for a time comprised between 1 hours and 6 hours.
From all of the above, it is clear to the skilled person that when preparing the compounds of formula (I) according to the aforementioned process, comprehensive of any variant thereof, optional functional groups within the starting materials or the intermediates thereof and which could give rise to unwanted side reactions, need to be properly protected according to conventional techniques. Likewise, the conversion of these latter into the free deprotected compounds may be carried out according to known procedures.
By analogy, any compound of formula (I) which is susceptible of being salified can be easily converted into the corresponding acid addition salt, by working in the presence of any pharmaceutically acceptable acid, for instance selected among those previously reported.
As it will be readily appreciated, if the compounds of formula (I) prepared according to the process described above are obtained as a mixture of isomers, their separation into the single isomers of formula (I), according to conventional techniques, is also within the scope of the present invention.
Conventional techniques for racemate resolution include, for instance, partitioned crystallization of diastereoisomeric salt derivatives or preparative chiral HPLC.

## PHARMACOLOGY

The compounds of formula (I) are active as protein kinase inhibitors and are therefore useful, for instance, to restrict the unregulated proliferation of tumor cells.
in therapy, they may be used in the treatment of various tumors, such as those formerly reported, as well as in the treatment of other cell proliferative disorders such as psoriasis, vascular smooth cell proliferation associated with atherosclerosis and post-surgical stenosis and restenosis and in the treatment of Alzheimer's disease.
The inhibiting activity of putative Cdc7 inhibitors and the potency of selected compounds is determined through a method of assay based on the use of Dowex resin capture technology. The assay consists of the transfer of radioactivity labeled phosphate moiety by the kinase to an acceptor substrate. The resulting 33P-labeled product is separated from unreacted tracer, transferred into a scintillation cocktail and light emitted is measured in a scintillation counter.

## Inhibition assay of Cdc7/Dbf4 activity

The inhibition assay of Cdc7/Dbf4 activity is performed according to the following protocol. The MCM2 substrate is trans-phosphorylated by the Cdc7/Dbf4 complex in the presence of ATP traced with $\gamma^{33}$-ATP. The reaction is stopped by addition of Dowex resin in the presence of formic acid. Dowex resin particles capture unreacted $\gamma^{33}$-ATP and drag it to the bottom of the well while $^{33}$P phosphorylated MCM2 substrate remains in solution. The supernatant is collected, transferred into Optiplate plates and the extent of substrate phosphorylation is evaluated by β counting.
The inhibition assay of Cdc7/Dbf4 activity was performed in 96 wells plate according to the following protocol.
To each well of the plate were added:

- 10 μl test compound (10 increasing concentrations in the nM to μM range to generate a dose-response curve). The solvent for test compounds contained 3% DMSO. (final concentration 1 %)

- 10 μl substrate MCM2 (6 μM final concentration), a mixture of cold ATP (2 μM final concentration) and radioactive ATP (1/5000 molar ratio with cold ATP).

- 10 μl enzyme (Cdc7/Dbf4, 2 nM final concentration) that started the reaction. The buffer of the reaction consisted in 50 mM HEPES pH 7.9 containing 15 mM $MgCl_2$, 2 mM DTT, 3 μM $NaVO_3$, 2mM glycerophosphate and 0.2mg/ml BSA.

After incubation for 60 minutes at room temperature, the reaction was stopped by adding to each well 150 μl of Dowex resin In the presence of 150 mM formic acid. After another 60 min incubation, 50 μL of suspension were withdrawn and transferred Into 96-well OPTIPLATEs containing 150 μl of MicroScint 40 (Packard); after 5-10 minutes shaking the plates were read for 1 min in a Packard TOP-Count radioactivity reader.

IC50 determination: inhibitors were tested at different concentrations ranging from 0.0005 to 10 μM. Experimental data were analyzed by the computer program Assay Explorer using the four parameter logistic equation:

$$y = bottom+(top-bottom)/(1+10^{((logIC50-x)*slope)})$$

where x is the logarithm of the inhibitor concentration, y is the response; y starts at bottom and goes to top with a sigmoid shape.

The inhibiting activity of putative cdk/cyclin inhibitors and the potency of selected compounds may be determined through a method of assay based on the use of the SPA technology (Amersham Pharmacia Biotech).

The assay consists of the transfer of radioactivity labelled phosphate moiety by the kinase to a biotinylated substrate. The resulting 33P-labelled biotinylated product is allowed to bind to streptavidin-coated SPA beads (biotin capacity 130 pmol/mg), and light emitted was measured in a scintillation counter.

**Inhibition assay of cdk2/Cyclin A activity**

**Kinase reaction:** 4 μM in house biotinylated histone H1 (Sigma # H-5505) substrate, 10 μM ATP (0.1 microCI $P^{33}\gamma$-ATP), 1.1 nM Cyclin A/CDK2 complex, inhibitor in a final volume of 30 μl buffer (TRIS HCl 10 mM pH 7.5, $MgCl_2$ 10 mM, DTT 7.5 mM + 0.2 mg/ml BSA) were added to each well of a 96 U bottom. After incubation for 60 min at room temperature, the reaction was stopped by addition of 100 μl PBS buffer containing 32 mM EDTA, 500 μM cold ATP, 0.1% Triton X100 and 10mg/ml streptavidin coated SPA beads. After 20 min incubation, 110 μL of suspension were withdrawn and transferred Into 96-well OPTIPLATEs containing 100 μl of 5M CsCl. After 4 hours, the plates were read for 2 min in a Packard TOP-Count radioactivity reader.

**IC50 determination**: inhibitors were tested at different concentrations ranging from 0.0015 to 10 μM. Experimental data were analyzed by the computer program GraphPad Prizm using the four parameter logistic equation:

$$y = bottom+(top-bottom)/(1+10^{((logIC50-x)*slope)})$$

where x is the logarithm of the inhibitor concentration, y is the response; y starts at bottom and goes to top with a sigmoid shape.

**Ki calculation:**

**Experimental method**: Reaction was carried out in buffer (10 mM Tris, pH 7.5, 10 mM $MgCl_2$, 0.2 mg/ml BSA, 7.5 mM DTT) containing 3.7 nM enzyme, histone and ATP (constant ratio of cold/labeled ATP 1/3000). Reaction was stopped with EDTA and the substrate captured on phosphomembrane (Multiscreen 96 well plates from Millipore). After extensive washing, the multiscreen plates were read on a top counter. Control (time zero) for each ATP and histone concentrations was measured.

**Experimental design**: Reaction velocities are measured at four ATP, substrate (histone) and inhibitor concentrations. An 80-point concentration matrix was designed around the respective ATP and substrate Km values, and the inhibitor IC50 values (0.3, 1, 3, 9 fold the Km or IC50 values). A preliminary time course experiment In the absence of inhibitor and at the different ATP and substrate concentrations allows the selection of a single endpoint time (10 min) in the linear range of the reaction for the Ki determination experiment.

**Kinetic parameter estimates**: Kinetic parameters were estimated by simultaneous nonlinear least-square

regression using [Eq.1] (competitive inhibitor respect to ATP, random mechanism) using the complete data set (80 points):

$$v = \frac{Vm \bullet A \bullet B}{\alpha \bullet Ka \bullet Kb + \alpha \bullet Ka \bullet B + a \bullet Kb \bullet A + A \bullet B + \alpha \bullet \frac{Ka}{Ki} \bullet I \bullet (Kb + \frac{B}{\beta})} \quad \text{[Eq.1]}$$

where A=[ATP], B=[Substrate], I=[inhibitor], Vm= maximum velocity, Ka, Kb, Ki the dissociation constants of ATP, substrate and inhibitor respectively. α and β the cooperativity factor between substrate and ATP binding and substrate and inhibitor binding respectively.

As an example, some representative compounds of the invention were tested as formerly reported against Cdc7/Dbf4 and Cdk2, showing an inhibitory activity, expressed as IC50 (nM), as follows:

2-(2-aminopyrimidin-4-yl)-1,5,6,7-tetrahydro-4H-pyrrolo[3,2-c]pyridin-4-one hydrochloride IC50 Cdc7: 11 nM;
2-(2-aminopyrimid in-4-yl)-7-phenyl-1,5,6,7-tetrahydro-4H-pyrrolo[3,2-c]pyridin-4-one hydrochloride IC50 Cdc7: 15nM;
2-(2-phenylaminopyrimidin-4-yl)-1,5,6,7-tetrahydro-4H-pyrrolo[3,2-c]pyridin-4-one hydrochloride IC50 Cdc7: 7nM;
2-(9H-purin-6-yl)-1,5,6,7-tetrahydro-4H-pyrrolo[3,2-c]pyridin-4-one hydrochloride IC50 Cdc7: 200nM;
2-pyrimidin-4-yl-1,5,6,7-tetrahydro-4H-pyrrolo[3,2-c]pyridin-4-one hydrochloride IC50 Cdc7: 19nM;
7-phenyl-2-(9H-purin-6-yl)-1,5,6,7-tetrahydro-4H-pyrrolo[3,2-c]pyridin-4-one hydrochloride IC50 Cdc7: 90nM;
2-(2-aminopyrimidin-4-yl)-6,6-dimethyl-1,5,6,7-tetrahydro-4H-pyrrolo[3,2-c]pyridin-4-one hydrochloride IC50 Cdc7: 32nM;
7-phenyl-2-pyrimidin-4-yl-1,5,6,7-tetrahydro-4H-pyrroto[3,2-c]pyridin-4-one hydrochloride IC50 Cdc7: 32nM ;
2-(2-anilinopyrimidin-4-yl)-7-phenyl-1,5,6,7-tetrahydro-4H-pyrrolo[3,2-c]pyridin-4-one hydrochloride IC50 Cdc7: 29nM;
2-(2-aminopyrimidin-4-yl)-6-isopropyl-1,5,6,7-tetrahydro-4H-pyrrolo[3,2-c]pyridin-4-one IC50 Cdc7: 9nM;
2-(2-aminopyrimidin-4-yl)-7-methyl-1,5,6,7-tetrahydro-4H-pyrrolo[3,2-c]pyridin-4-one IC50 Cdc7: 8nM;
2-(2-aminopyrimidin-4-yl)-6-methyt-1,5,6,7-tetrahydro-4H-pyrrolo[3,2-c]pyridin-4-one IC50 Cdc7: 26nM;
2-(2-aminopyrimidin-4-yl)-7,7-dimethyl-1,5,6,7-tetrahydro-4H-pyrrolo[3,2-c]pyridin-4-one IC50 Cdc7: 2nM;
2-(2-aminopyrimidin-4-yl)-6-isobutyl-1,5,6,7-tetrahydro-4H-pyrrolo[3,2-c]pyridin-4-one IC50 Cdc7: 4nM;
ethyl 4-(4-oxo-4,5,6,7-tetrahydro-1H-pyrrolo[3,2-c]pyridin-2-yl)pyrimidin-2-ylcarbamate IC50 Cdc7: 80nM;
(7R or 7S)-2-(2-aminopyrimidin-4-yl)-7-methyl-1,5,6,7-tetrahydro-4H-pyrrolo[3,2-c]pyridin-4-one IC50 Cdc7: 5nM;
(7R or 7S)-2-(2-am(nopyrirnldin-4-yl)-7-methyl-1,5,6,7-tetrahydro-4H-pyrrolo[3,2-c]pyridin-4-on IC50 Cdc7: 4nM;
2-(2-aminopyrimid)in-4-yl)-3-iodo-1,5,6,7-tetrahydro-4H-pyrrolo[3,2-c]pyridin-4-one IC50 Cdc7: 4nM;
2-(2-aminopyrimidin-4-yl)-7,7-diethyl-1,5,6,7-tetrahydro-4H-pyrrolo[3,2-c]pyridin-4-one IC50 Cdc7: 7nM;
2-{2-[(2-furylmethyl)amino]pyrimidin-4-yl}-1,5,6,7-tetrahydro-4H-pyrrolo[3,2-c]pyridin-4-one IC50 Cdc7: 74nM;
N-[4-(4-oxo-4,5,6,7-tetrahydro-1H-pyrrolo[3,2-c]pyrldin-2-yl)pyrimidin-2-yl]benzamide IC50 Cdc7: 300nM;
2-(2-aminopyrimidin-4-yl)-7-isopropyl-1,5,6,7-tetrahydro-4H-pyrrolo[3,2-c]pyridin-4-one IC50 Cdc7: 8nM;
2-[2-(benzylamino)pyrimidin-4-yl]-1,5,6,7-tetrahydro-4H-pyrrolo[3,2-c]pyridin-4-one IC50 Cdc7: 270nM;
2-[2-(propylamino)pyrimidin-4-yl]-1,5,6,7-tetrahydro-4H-pyrrolo[3,2-c]pyridin-4-one IC50 Cdc7: 310nM; IC50 Cdk2: 35nM;
2-[2-(Isobutylamino)pyrimidin-4-yl]-1,5,6,7-tetrahydro-4H-pyrrolo[3,2-c]pyridin-4-one IC50 Cdk2: 50nM;
2-{2-[(cyclohexylmethyl)amino]pyrimidin-4-yl}-1,5,6,7-tetrahydro-4H-pyrrolo[3,2-c]pyridin-4-one IC50 Cdc7: 680nM; IC50 Cdk2: 220nM;
2-{2-[(2-furylmethyl)amino]pyrimidin-4-yl}-1,5,6,7-tetrahydro-4H-pyrrolo[3,2-c]pyridin-4-one IC50 Cdc7: 87nM; IC50 Cdk2: 80nM and
N-[4-({[4-(4-oxo-4,5,6,7-tetrahydro-1H-pyrrolo[3,2-c]pyridln-2-yl)pyrimidin-2-yl]amino}methyl)phenyl]acetamide trifluoroacetate
(C50 Cdc7: 300nM; IC50 Cdk2: 20nM.

The compounds of the present invention can be administered either as single agents or, alternatively, in combination with known anticancer treatments such as radiation therapy or chemotherapy regimen in combination with cytostatic or cytotoxic agents, antibiotic-type agents, alkylating agents, antimetabolite agents, hormonal agents, immunological agents, interferon-type agents, cyclooxygenase Inhibitors (e.g. COX-2 Inhibitors), matrixmetalloprotease inhibitors, telomerase inhibitors, tyrosine kinase inhibitors, anti-growth factor receptor agents, anti-HER agents, anti-EGFR agents, anti-anglogenesis agents (e.g. angiogenesis inhibitors), farnesyl transferase inhibitors, ras-raf signal transduction pathway inhibitors, cell cycle Inhibitors, other cdks inhibitors, tubulin binding agents, topoisomerase I inhibitors and

topoisomerase II inhibitors.

If formulated as a fixed dose, such combination products employ the compounds of this invention within the dosage range described below and the other pharmaceutically active agent within the approved dosage range.

Compounds of formula (I) may be used sequentially with known anticancer agents when a combination formulation is inappropriate.

The compounds of formula (I) of the present invention, suitable for administration to a mammal, e.g., to humans, can be administered by the usual routes and the dosage level depends upon the age, weight, conditions of the patient and administration route.

For example, a suitable dosage adopted for oral administration of a compound of formula (I) may range from 10 to 500 mg per dose, from 1 to 5 times daily. The compounds of the invention can be administered in a variety of dosage forms, e.g., orally, in the form tablets, capsules, sugar or film coated tablets, liquid solutions or suspensions; rectally in the form suppositories; parenterally, e.g., intramuscularly, or through intravenous and/or intrathecal and/or intraspinal injection or infusion.

The present invention also includes pharmaceutical compositions comprising a compound of formula (I) or a pharmaceutically acceptable salt thereof in association with a pharmaceutically acceptable excipient, which may be a carrier or a diluent.

The pharmaceutical compositions containing the compounds of the invention are usually prepared following conventional methods and are administered In a suitable pharmaceutical form.

For example, the solid oral forms may contain, together with the active compound, diluents, e.g., lactose, dextrose saccharose, sucrose, cellulose, corn starch or potato starch; lubricants, e.g., silica, talc, stearic acid, magnesium or calcium stearate, and/or polyethylene glycols; binding agents, e.g., starches, arabic gum, gelatine methylcellulose, carboxymethylcellulose or polyvinyl pyrrolidone; disintegrating agents, e.g., starch, alginic acid, alginates or sodium starch glycolate; effervescing mixtures; dyestuffs; sweeteners; wetting agents such as lecithin, polysorbates, laurylsulphates; and, in general, non-toxic and pharmacologically inactive substances used in pharmaceutical formulations. These pharmaceutical preparations may be manufactured in known manner, for example, by means of mixing, granulating, tabletting, sugar-coating, or film-coating processes.

The liquid dispersions for oral administration may be, e.g., syrups, emulsions and suspensions.

As an example, the syrups may contain, as carrier, saccharose or saccharose with glycerine and/or mannitol and sorbitol.

The suspensions and the emulsions may contain, as examples of carriers, natural gum, agar, sodium alginate, pectin, methylcellulose, carboxymethylcellulose, or polyvinyl alcohol.

The suspension or solutions for Intramuscular injections may contain, together with the active compound, a pharmaceutically acceptable carrier, e.g., sterile water, olive oil, ethyl oleate, glycols, e.g., propylene glycol and, if desired, a suitable amount of lidocaine hydrochloride.

The solutions for intravenous Injections or infusions may contain, as a carrier, sterile water or preferably they may be In the form of sterile, aqueous, isotonic, saline solutions or they may contain propylene glycol as a carrier.

The suppositories may contain, together with the active compound, a pharmaceutically acceptable carrier, e.g., cocoa butter, polyethylene glycol, a polyoxyethylene sorbitan fatty acid ester surfactant or lecithin.

With the aim to better illustrate the present invention, the following examples are now given.

## General methods

Flash Chromatography was performed on silica gel (Merck grade 9395, 60A). HPLC was performed on Waters X Terra RP 18 (4,6 x 50 mm, 3.5 μm) column using a Waters 2790 HPLC system equipped with a 996 Waters PDA detector and Micromass mod. ZQ single quadrupole mass spectrometer, equipped with an electrospray (ESI) ion source. Mobile phase A was ammonium acetate 5 mM buffer (pH 5.5 with acetic acid/acetonitrile 95:5), and Mobile phase B was $H_2O$/acetonitrile (5:95). Gradient from 10 to 90% B in 8 minutes, hold 90% B 2 minutes. UV detection at 220 nm and 254 nm. Flow rate 1 ml/min. Injection volume 10 μl. Full scan, mass range from 100 to 800 amu. Capillary voltage was 2.5 KV; source temp. was 120°C; cone was 10 V. Retention times (HPLC r.t.) are given in minutes at 220 nm or at 254 nm. Mass are given as m/z ratio.

When necessary, compounds have been purified by preparative HPLC on a Waters Symmetry C18 (19 x 50 mm, 5 um) column using a Waters preparative HPLC 600 equipped with a 996 Waters PDA detector and a Micromass mod. ZMD single quadrupole mass spectrometer, electron spray ionization, positive mode. Mobile phase A was water 0.01% TFA, and Mobile phase B was acetonitrile. Gradient from 10 to 90% B in 8 min, hold 90% B 2 min. Flow rate 20 ml/min.

1H-NMR spectrometry was performed on a Mercury VX 400 operating at 400.45 MHz equipped with a 5 mm double resonance probe [1 H (15N-31 P) ID_PFG Varian].

The compounds of formula (I), having an asymmetric carbon atom and obtained as racemic mixture, were

resolved by HPLC separation on chiral columns. In particular, for example, preparative columns CHIRALPACK® AD, CHIRALPACK® AS, CHIRALCELL®OJ can be used.

**Example 1**

**2-bromo-1-pyrimidin-4-ylethanone hydrobromide**

The title compound was prepared by working as described in J. Med. Chem. 1992, 35, 3288. Onto a stirred solution of pyrimidine (2.5 g, 31.2 mmols) and acetaldehyde (10.8 mL, 192 mmols) in dichloromethane (190 mL) at about 0°C, 3.4 M sulphuric acid (15.6 mL) was added dropwise. The solution was cooled to -5°C and, from two distinct dropping funnels, two solutions were simultaneously dropped therein in about 30 minutes: an 80% solution of tert-butylhydroperoxide in di-tert-butylperoxide/water (23.4 mL) and a solution of ferrous sulphate heptahydrate (52.2 g) in 100 mL of water. After addition, the reaction mixture was stirred at 0°C for 2.5 hours, then the phases were separated and the aqueous phase was extracted with dichloromethane (2 x 150 mL). The joined organic phases were washed with a 10% aqueous sodium iodide solution, with $Na_2S_2O_5$, (10% aqueous solution) and with brine and then were dried over $Na_2SO_4$- Upon concentration, the obtained yellow solid was taken up with petroleum ether and filtered. After drying, the title compound was obtained as a brownish solid (0.87 g, Y=23 %).
$^1$H NMR (DMSO-$d_6$ / 300 MHz) δ ppm 5.0 (s, 2H), 7.98 (d, 1 H), 9.12 (d, 1H), 9.42 (s, 1H). bs, 1H).

**Example 2**

**1-(2-aminopyrimidin-4-yl)-2-bromoethanone hydrobromide**

The title compound (a) was prepared by working as described in J. Het. Chem. 1985, 22, 1723.

A mixture of 3,3-dimethoxy-2-butanone (25 g, 189.16 mmol) and N,N-dimethylformamide dimethylacetal (22.5 g, 189.16 mmol) were stirred at 110°C for 30 hours and then distilled (115°C, 1 mmHg) thus obtaining 1-(dimethylamino)-4,4-dimethoxypent-1-en-3-one, as a yellow solid (27.3 g, 146 mmol, 77%).

Onto a solution of sodium (3.48 g, 151.67 mmol) In anhydrous ethanol (400 mL), solid guanidine hydrochloride (14.5 g, 151.67 mmol) was added at r.t., to give a white suspension into which a solution of 1-(dimethylamino)-4,4-dimethoxypent-1-en-3-one (28.4 g, 151.67 mmol) in anhydrous ethanol (50 mL) was added. The mixture was refluxed for 19 hours. After cooling, the precipitate was filtered and washed with ethanol and with plenty of water, thus obtaining a white solid (8.56 g). The ethanolic solutions were concentrated to dryness, taken up with boiling ethyl acetate (1000 mL), filtered while hot and then cooled to yield a second crop. Total amount of 4-(1,1-dimethoxyethyl)pyrimidin-2-amine: 17.66 g, 63.5%. A solution of the said amine (17.5 g, 95.5 mmol) in formic acid was stirred at r.t. for 6 hours and concentrated to dryness and the residue was stirred in ethanol (50 mL) and then filtered thus obtaining 1-(2-aminopyrimidin-4-yl)ethanone (9.2 g, 70%). To a solution of 1-(2-aminopyrimidin-4-yl)ethanone (412 mg, 3 mmol) in glacial acetic acid (1 mL) and 48% aq. HBr (0.3 mL), bromine (0.153 mL) in acetic acid (0.4 mL) was added and the resulting orange solution was stirred at r.t. for 15 hours. After diluting with ethyl acetate (15 mL) the precipitate was filtered and washed with ethyl acetate thus affording the title compound as a whitish solid (580 mg, 65%).
$^1$H NMR (DMSO-$d_6$ / 300 MHz) δ ppm: 4.9 (s, 2H), 7.0 (d, 2H), 8.5 (d, 2H). s, 6H), 2.49 (bs, 2H), 3.13 (bs, 2H), 8.13 (bs, 1H).

**Example 3**

**2-bromo-1-(9H-purin-6-yl)ethanone**

A solution of 6-(1-ethoxyvinyl)-9-tetrahydro-2H-pyran-2-yl-9H-purine (430 mg, 1.57 mmols), being prepared as described in Tetrahedron, 53 (6), 2291-2302, (1997), in tetrahydrofuran (24 ml) and water (1.5 ml) was treated with N-bromo-succinimide (NBS, 280 mg, 1.57 mmols) and kept at room temperature for 15 minutes. The solution was evaporated under reduced pressure, taken up with water and filtered to obtain the title compound (312 mg, Y=82%) as a yellow solid.
$^1$H NMR (DMSO-$d_6$ / 300 MHz) δ ppm 5.12 (s, 2H), 8.85 (s, 1 H), 9.14 (s, 1H)

**Example 4**

**1-(2-phenylaminopyrimidin-4-yl)-2-chloroethanone**

To a solution of sodium (614 mg, 26.7 mmol) in anhydrous ethanol (70 mL), phenylguanidine (4.43 g, 13.35 mmol) was added followed by a solution of 1-(dimethylamino)-4,4-dimethoxypent-1-en-3-one (5 g, 26.7 mmol), obtained

as previously described, in anhydrous ethanol (20 mL). The suspension was refluxed for 20 hours, then 2/3 of the solvent were removed and water (250 mL) was added. The precipitate was extracted with ethyl acetate and the organic phase was washed with a sodium dihydrogenphosphate solution, with brine, dried over sodium sulphate and concentrated to yield the desired ketal (4.3 g). The ketal (4.2 g, 16.19 mmol) was dissolved in 88% formic acid (25 mL) and stirred at r.t. for 2.5 hours. The reaction mixture was diluted with water (200 mL), the precipitate was filtered and washed with abundant water. 1-(2-Phenylaminopyrlmldln-4-yl)-ethanone was thus isolated as a yellow solid (3.1 g).

This ketone (1.3 g, 6.1 mmol) was dissolved in dichloromethane (40 mL), then TEA (5.1 mL, 36.6 mmol) and tert-butyl-dimethyl-trifluoro methansulphonate (4.2 mL, 18.3 mmol) were added. The orange solution was stirred overnight, then diluted with more dichloromethane (150 mL), washed twice with a 5% sodium hydrogencarbonate solution (50 mL), with water, with brine, then dried over sodium sulphate and concentrated to give the bis-silylated derivative (2.67 g). To half of the material (1.32 g, 2.94 mmol) dissolved in tetrahydrofuran (THF, 25 mL) and cooled to 0°C, a solution of NBS (0.549 g, 3.09 mmol) in THF (10 mL) was added dropwise in 5 minutes. The reaction mixture was stirred at 0°C for 1 hour, then 2 N HCl was added and the reaction mixture was stirred for 24 hours at r.t. The crude was purified by flash chromatography (eluant: hexane/ethyl acetate 5:1) to yield 0.325 g of title compound.
$^1$H NMR (DMSO-$d_6$ / 300 MHz) δ ppm: 4.9 (s, 2H), 6.7 (m, 1H), 6.9 (d, 1H), 7.0 (m, 2H), 7.4 (d, 2H), 8.4 (d, 1 H), 9.6 (s, 1H).

## Example 5

### Preparation of 6,6-dimethyl-2,4-dioxopiperidine

A solution of ethyl 3-methylbut-2-enoate (1 g, 7.8 mmol) in anhydrous ethanol (12 mL) was cooled to -20 °C and saturated with gaseous ammonia. The tube was sealed and kept at 90°C for 24 hours. The reaction was cooled to room temperature, bubbled with nitrogen to eliminate the residual ammonia and treated with a 4 N solution of HCl In dioxane (1.9 mL). After 30 minute stirring, the mixture was evaporated under reduced pressure to give ethyl 3-amlno-3-methylbutanoate hydrochloride as a grey solid (1.19 g, Y=84%).
$^1$H NMR ($CDCl_3$ / 400 MHz) δ ppm 1.2 (t, 3H), 1.26 (s, 6H), 2.65 (s, 2H), 4.1 (q, 2H), 8.27 (bs, 3H).

Ethyl 3-amino-3-methylbutanoate hydrochloride (0.87 g, 4.79 mmol) was suspended on methylene chloride (12 mL) and triethylamine (1.4 mL, 2.1 eq.). The mixture was cooled to 0°C and treated dropwise with ethyl 3-chloro-3-oxopropanoate (0.64 mL, 1.05 eq.). The reaction was kept at room temperature for 2 hours, diluted with methylene chloride, washed with 1 N HCl and then with 5% $NaHCO_3$, dried over $Na_2SO_4$ and evaporated to dryness to obtain ethyl 3-[(3-ethoxy-3-oxopropanoyl)amino]-3-methylbutanoate (1.2g, Y=97%) as a red oil.
$^1$H NMR (DMSO-$d_6$ / 300 MHz) δ ppm 1.11-1.21 (m, 6H), 1.29 (s, 6H), 2.71 (s, 2H), 3.14 (s, 2H), 3.95-4.15 (m, 4H), 7.75 (bs, 1H).

To a solution of sodium ethoxide, obtained from sodium metal (0.122 g, 5.55 mmol) in anhydrous ethanol (7 mL), a solution of ethyl 3-[(3-ethoxy-3-oxopropanoyl)amino]-3-methylbutanoate (1.2 g, 4.62 mmol) in dry toluene (7 mL) was added dropwise at room temperature, under stirring. The reaction mixture was heated at 80°C for 2 hours then it was concentrated to reduced volume and the residue was dissolved in toluene (15 mL). The organic phase was extracted with water (40 mL), the aqueous phase was acidified to pH 2-3 with 1 N HCl and extracted with ethyl acetate (4 x 50 mL). The organic phase was washed with brine, dried over anhydrous sodium sulphate and concentrated to give ethyl 6,6-dimethyl-2,4-dioxopiperidine-3-carboxylate as a yellow solid (0.7 g, Y=71%) which was used for the next step without further purification.

Ethyl 6,6-dimethyl-2,4-dioxopiperidine-3-carboxylate (0.69 g, 3.23 mmol) was dissolved in acetonitrile containing 1% of water (15 mL) and the resulting solution was refluxed for 2 hours. After evaporating to dryness, the crude material was suspended in isopropyl ether, kept under vigorous stirring and filtered to give the title compound (387 mg, Y=85%) as a light brown solid.
$^1$H NMR (DMSO-$d_6$ / 300 MHz) δ ppm 1.18 (s, 6H), 2.49 (bs, 2H), 3.13 (bs, 2H), 8.13 (bs, 1H).

## Example 6

### Preparation of 5-phenylpiperidine-2,4-dione

Ethyl cyano(phenyl)acetate (14.9 g, 78.83 mmol) was dissolved in absolute ethanol (400 mL) containing 37% hydrochloric acid (40 mL). The solution was treated with 10% Pd-C (2 g) and kept under hydrogen (40 psi) in a Parr apparatus for 24 hours. The resulting mixture was filtered to remove the catalyst and evaporated to dryness under reduced pressure. The residue was taken up with ethyl acetate, kept under vigorous stirring for 15 minutes and filtered. Obtained ethyl 3-amino-2-phenylpropanoate hydrochloride (11 g, Y=60%).
$^1$H NMR (DMSO-$d_6$ / 300 MHz) δ ppm 1.11 (t, 3H), 3.05 (dd, 1H. J=12.9, 6.15), 3.42 (dd, 1H, J=12.9, 8.79), 4.07 (m, 3H), 7.35 (m, 5H), 8.15 (br, 3H)

Ethyl 3-amino-2-phenylpropanoate hydrochloride (4.38 g, 19.13 mmol) was suspended in methylene chloride (80 mL) and triethylamine (5.86 mL, 2.2 eq.). The mixture was cooled to 0°C and treated dropwise with ethyl 3-chloro-3-oxopropanoate (2.69 mL, 1.1 eq.). The reaction was kept at room temperature for one hour, diluted with methylene chloride, washed with 1 N HCl and then with 5% $NaHCO_3$, dried over $Na_2SO_4$ and evaporated to dryness. The crude material was chromatographed on silica gel, eluting with hexane/ethyl acetate 1/1, to give 3-(2-ethoxycarbonyl-acetylamino)-2-phenyl-propionic acid ethyl ester (4.24 g, Y=72%) as an oil.
$^1$H NMR ($CDCl_3$/ 300 MHz) δ ppm 1.21 (t, 3H), 1.27 (t, 3H), 3.26 (s, 2H), 3.73 (m, 2H), 3.89 (dd, 1H, J=6.16, 8.50), 4.17 (m, 4H), 7.29 (m, 6H).

Sodium (380 mg, 16.52 mmol) was dissolved in anhydrous ethanol (13 mL) and the resulting solution was treated dropwise with 3-(2-ethoxycarbonyl-acetylamino)-2-phenyl-propionic acid ethyl ester (4.23 g, 13.76 mmol) dissolved in anhydrous toluene (35 mL). The reaction was kept at 80°C for 1.5 hours. After cooling, the mixture was extracted with water. The aqueous extracts were acidified with 2 N HCl, extracted with ethyl acetate and the organic layers were collected, dried over $Na_2SO_4$ and evaporated to dryness to obtain ethyl 2,4-dioxo-5-phenylpiperidine-3-carboxylate (1.73 g, Y=48%) which was used for the next step without further purification. Ethyl 2,4-dioxo-5-phenylpiperidine-3-carboxylate (1.73 g, 6.63 mmol) was dissolved in acetonitrile containing 1% of water (30 mL) and the resulting solution was refluxed for 2 hours. After evaporating to dryness, the crude material was chromatographed on silica gel, eluting with methylene chloride/methanol 92/8, to give the title compound (780 mg, Y=62%) as a solid.
$^1$H NMR (DMSO-$d_6$ / 300 MHz) δ ppm 3.25 (d, 1H, J=18.75) 3.42-3.70 (m,2H), 3.61 (d, 1H, J=18.75), 3.81 (dd, 1 H, J=5.57, 9.67), 7.26 (m, 5H), 8.20 (bs, 1H).

## Example 7

### Preparation of ethyl 2-(aminomethyl)-3-methylbutanoate hydrochloride

Ethyl 2-cyano-3-methylbut-2-enoate (5.0 g, 32.6 mmol) was dissolved in 320 mL of absolute EtOH. 700 mg of $PtO_2$ and 12 mL of 4M HCl were added. The reaction mixture was hydrogenated at room temperature for 5 hours (30 psi). Filtration on a celite pad and evaporation of the solvent afforded crude title compound (quantitative yleid). 1H NMR (400 MHz, DMSO-D6) δ ppm 0.90 (d, *J*=6.83 Hz, 3 H) 0.93 (d, *J*=6.83 Hz, 3 H) 1.24 (t, *J*=7.13 Hz, 3 H) 1.92 - 2.06 (m, 1 H) 2.53 - 2.60 (m, 1 H) 2.84 - 3.17 (m, 2 H) 4.05 - 4.24 (m, 2 H) 7.84 (s, 3 H)
ESI (+) MS: m/z 160 (MH+).

By working in an analogous way and by starting from the suitable cyano derivative, the following compounds were also prepared:

### ethyl 2-(aminomethyl)-3-methylpentanoate hydrochloride

$^1$H NMR (DMSO-$d_6$ / 400 MHz) δ ppm 0.80 (m, 6 H) 1.23-1.40 (2 m, 5 H) 1.76 (m, 1 H) 2.69 (m, 1 H) 2.89 (m, 1H), 3.09 (m, 1H) 4.14 (m, 2H) 7.82 (s, 3H)
ESI (+) MS: m/z 174 (MH+).

### ethyl 1-(aminomethyl)cyclopropanecarboxylate hydrochloride

ESI (+) MS: m/z 144 (MH+).

## Example 8

### Preparation of ethyl 2-{[(3-ethoxy-3-oxopropanoyl)amino]methyl}-3-methylbutanoate

Crude ethyl 2-(aminomethyl)-3-methylbutanoate hydrochloride was dissolved in 200 mL of dry DCM and DIPEA was added (14 mL, 2.5 eq). After cooling to 0°C, ethyl 3-chloro-3-oxopropanoate was added (6.3 mL, 35.4 mmol). After stirring at room temperature overnight, the reaction mixture was diluted with DCM and washed with aq. $KHSO_4$ 5% (x2), aq. $NaHCO_3$ sat. sol. (x2) and brine. The organic layer was dried over $Na_2SO_4$, filtered and evaporated to dryness. Column chromatography (hexane/EtOAc=7/3 -> 1/1) afforded 8.35 g (30.55 mmol, 93.4% yield) of target product.
1H NMR (400 MHz, DMSO-D6) δ ppm 0.89 (d, *J*=6.82 Hz, 3 H) 0.93 (d, *J*=6.83 Hz, 3 H) 1.20 (m, 6 H) 1.82 - 1.90 (m, 1 H) 2.35 (m, 1 H) 3.19 - 3.33 (2 m, 4 H) 4.06 (m, 4 H) 8.11 (t, *J*=5.12 Hz, 1 H)
ESI (+) MS: m/z 274 (MH+).

By working in an analogous way and by starting from the suitable hydrochloride derivative, the following compounds were prepared:

**ethyl 2-{[(3-ethoxy-3-oxopropanoyl)amino]methyl}-3-methylpentanoate**

$^1$H NMR (DMSO-$d_6$ / 400 MHz) δ ppm 0.90 (m, 6 H) 1.19-1.65 (3 m, 9 H) 2.47 (m, 1 H) 3.20 (m, 4H), 4.08 (m, 4H), 8.09 (m, 1H)
ESI (+) MS: m/z 288 (MH+).

**ethyl 1-{[(3-ethoxy-3-oxopropanoyl)amino]methyl}cyclopropanecarboxylate**

ESI (+) MS: m/z 258 (MH+).

**Example 9**

**Preparation of 5-isopropylpiperidine-2,4-dione**

Crude ethyl 2-{[(3-ethoxy-3-oxopropanoyl)amino]methyl}-3-methylbutanoate (8.35 g,30.55 mmol) was dissolved in 215 mL of dry toluene and heated to 100°C. 6.9 mL of sodium methoxide 30 wt.% solution In methanol were added (36 mmol) and the reaction mixture was refluxed for 4 hours. After cooling at room temperature, the organic phase was washed with water (x2). The aqueous layers were collected, acidified (10% HCl) and extracted with DCM (x4). The organic layers were collected and evaporated to dryness. The crude was treated with 250 mL of 10% AcOH In water and refluxed for 3 hours. The reaction mixture was neutralized with $NaHC0_3$ (- pH 7) and extracted with DCM (x5). The organic layers were collected, dried ($Na_2SO_4$), filtered and evaporated to dryness. Column chromatography (DCM/EtOH=97/3) afforded 2.35 g of target product (15.14 mmol, 49.6% yield).
1 H NMR (400 MHz, DMSO-D6) δ ppm 0.85 (d, *J*=6.83 Hz, 3 H) 0.94 (d, *J*=6.95 Hz, 3 H) 2.07 - 2.17 (m, 1 H) 2.25 - 2.33 (m, 1 H) 3.09 - 3.41 (m, 4 H) 8.03 (s, 1 H)
ESI (+) MS: m/z 156 (MH+).

By working In an analogous way and by starting from the suitable aminoester derivative, the following compounds were prepared:

**5-sec-butylpiperidine-2,4-dione**

$^1$H NMR (DMSO-$d_6$ / 400 MHz) δ ppm 0.87 (m, 6 H) 1.36 (m, 2 H) 1.95 (m, 1 H) 2.35 (m, 1 H) 3.34 (m, 4H), 8.02 (s, 1 H)
ESI (+) MS: m/z 170 (MH+).

**5-azaspiro[2.5]octane-6,8-dione**

1 H NMR (400 MHz, DMSO-D6) δ ppm 0.95 - 1.02 (m, 2 H) 1.09 - 1.15 (m, 2 H) 3.33 (s, 2 H) 3.42 (s, 2 H) 8.22 (s, 1 H)
ESI (+) MS: m/z 140 (MH+).

**5,5-diethylpiperidine-2,4-dione**

1H NMR (400 MHz, DMSO-D6) δ ppm 0.77 (t, *J*=7.56 Hz, 6 H) 1.46 (q, *J*=7.68 Hz, 4 H) 3.23 (d, *J*=3.78 Hz, 2 H) 3.26 (s, 2 H) 7.98 (s, 1 H)

**Example 10**

**6-Benzylplperldine-2,4-dione**

A mixture of beta-homophenylalanine (9.1 g, 50.94 mmol), di-tert-butyl dicarbonate (12.2 g, 56.1 mmol), dioxane (180 mL), water (18 mL) and triethylamine (8.5 mL) was stirred at RT overnight. After concentration and multiple strippings with toluene, 3-[(tert-butoxycarbonyl)amino]-4-phanylbutanoic acid was obtained as an oil and used directly In the next step. It was dissolved In dry dichloromethane (370 mL), Meldrum acid (8.1 g, 56.1 mmol) and DMAP (9.7 g, 79 mmol) were added to It, the mixture was cooled to -5°C and dicyclohexylcarbodiimide (12.6 g, 61 mmol) was added. After addition the reaction mixture was kept in refrigerator overnight. The precipitate was filtered off and washed with dichloromethane. The filtrate was diluted with ethylacetate, washed in sequence with 10% aq $KHSO_4$, water, brine then concentrated to yield crude tert-butyl 1-benzyl-3-(2,2-dimethyl-4,6-dloxo-1,3-dioxan-5-yl)-3-oxopropylcarbamate that was dissolved in ethylacetate (250 mL) and refluxed 2 h. After concentration and treatment with diisopropylether the

crystallized compound was filtered and washed to give tert-butyl 2-benzyl-4,6-dloxopiperidine-1-carboxylate as a white powder in 75% overall yield.

The t-butoxycarbonyl group could be removed by acidic treatment (4M HCl in dioxane) at RT. 1H NMR (400 MHz, DMSO-D6) δ ppm 2.32 (dd, *J*=15.73, 8.17 Hz, 1 H) 2.42 (dd, *J*=16.34, 4.76 Hz, 1 H) 2.66 - 2.74 (m, 1 H) 2.87 - 3.02 (m, 2 H) 3.25 - 3.40 (m, 1 H) 3.84 - 3.93 (m, 1 H) 7.20 - 7.36 (m, 5 H) 8.14 (s, 1 H).

By working in an analogous way the following compounds were also obtained:

**6-isopropylpiperidine-2,4-dione**

ESI (+) MS: m/z 156 (MH+).

**6-methylplperidine-2,4-dione**

ESI (+) MS: m/z 128 (MH+).

**5,5-dimethylpiperidine-2,4-dione**

1H NMR (300 MHz, DMSO-D6) δ ppm 1.0 (s, 6 H) 3.15 (s, 2 H) 3.25 (s, 2 H) 8.0 (s, 1 H).

**6-(2-phenylethyl)piperidine-2,4-dione**

ESI (+) MS: m/z 218 (MH+).

**Example 11**

**Preparation of 5-benzylpiperidine-2,4-dione**

To a solution of tert-butyl 2,4-dioxopiperidine-1-carboxylate (324 mg, 1.5 mmol) in dry THF (10 mL), cooled to -20°C under nitrogen, lithium bis(trimethylsilyl)amide (LiHMDS) (4 mL of 1 M solution in THF) was added dropwise. After 20 min stirring, 3.0 eq of benzyl bromide were added and the solution was stirred at -20°C for 2 hours. The reaction mixture was poured into 5% aq $KHSO_4$ and extracted with DCM (x2). To the collected (200 mL) organic layers, 20 mL of TFA were added and the resulting solution was stirred at room temperature for 2 hours. After evaporation, the residue was purified by column chromatography (hexane/EtOAc 1:2) affording 150 mg of target product (0.74 mmol, 49%).
1 H NMR (400 MHz, DMSO-D6) δ ppm 2.81 (m, 1 H), 3.12 (m, 2 H) 3.34 (m, 4 H), 7.23 - 7.30 (m, 5H), 7.99 (s, 1 H)
ESI (+) MS: m/z 204 (MH+).

By working in an analogous way and by using the suitable alkyl halide, the following compounds were prepared:

**5-isobutylpiperidine-2,4-dione**

1H NMR (400 MHz, DMSO-D6) δ ppm 0.88 (m, 6H), 1.16 (m, 1 H), 1.53 (m, 1 H), 1.61 (m, 1 H), 3.08 (m, 1 H) 3.20 - 3.40 (m, 4 H), 8.03 (s, 1 H)
ESI (+) MS: m/z 170 (MH+).

**5-ethylpiperldine-2,4-dione**

1 H NMR (400 MHz, DMSO-D6) δ ppm 0.89 (t, *J*=7.56, 3H), 1.35 (m, 1H), 1.69 (m, 1H), 2.39 (m, 1H), 3.14 - 3.38 (m, 4 H), 8.05 (s, 1 H)
ESI (+) MS: m/z 142 (MH+).

**Example 12**

**Preparation of tert-butyl 5-ethyl-2,4-dioxopiperldine-1-carboxylate**

To a solution of tert-butyl 2,4-dioxopiperidine-1-carboxylate (1.92 g, 9.0 mmol), in dry THF (65 mL) and cooled to -20°C under nitrogen, lithium bis(trimethylsilyl)amide (LiHMDS) (27 mL of 1 M solution in THF) was added dropwise. After 20 min stirring, 2.53 mL (4.9 g, 31.3 mmol) of lodoethane were added and the solution was stirred at -20°C for 2 hours. The reaction mixture was poured in 5% aq $KHSO_4$ and extracted with DCM (x2). The collected organic layers were washed with water, dried over $Na_2SO_4$ and evaporated to dryness. The residue was purified by column chromatography (n-Hexane/EtOAc 1:1) affording 1.4 g of target product (5.8 mmol, 64%).
ESI (+) MS: m/z 242 (MH+).

By working In an analogous way and by using 1-lodo-3-methylbutane, the following compound was prepared:

**tert-butyl 5-isobutyl-2,4-dioxopiperidine-1-carboxylate**

ESI (+) MS: m/z 270 (MH+).

**Example 13**

**2-pyrimidin-4-yl-1,5,6,7-tetrahydro-4H-pyrrolo[3,2-c]pyridin-4-one hydrochloride**

2-Bromo-1-pyrimidin-4-ylethanone hydrobromide (67 mg, 0.239 mmols), piperidine-2,4-dione (50 mg, 0.358 mmols) and ammonium acetate (74 mg, 0.957 mmols) were dissolved in anhydrous ethanol (1 mL) and stirred at r.t. overnight. The reaction mixture was concentrated to dryness under reduced pressure and the residue was taken up with water (1 mL) and filtered; the solid was washed with cold water and dried.

To the obtained brown solid (30 mg) dissolved In MeOH (15 mL), 4N HCl in dioxane (0.5 mL) was added and the mixture was stirred for 30 minutes and then concentrated under reduced pressure to half of the volume.

The obtained precipitate was filtered, washed with ethyl acetate and dried to give the title compound as a yellow solid (31 mg, Y=52%).
$^1$H NMR (DMSO-$d_6$ / 400 MHz) δ ppm 2.87 (t, 2H, J=6.83), 3.44 (t, 2H, J=6.83), 7.19 (bs, 1H). 7.37 (s, 1H), 7.89 (d, 1H, J=5.85), 8.68 (d, 1H, J=5.85), 9,10 (s, 1 H)

By working in an analogous way the following compounds were also obtained:

**2-(2-aminopyrimidin-4-yl)-1,5,6,7-tetrahydro-4H-pyrrolo[3,2-c]pyridin-4-one hydrochloride**

$^1$H NMR (DMSO-$d_6$ / 400 MHz) δ ppm 2.91 (t, 2H, J=6.71), 3.36 (t, 2H, J=6.71), 7.27 (d, 1H, J=6.70), 7.29 (bs, 1H), 7.46 (s, 1H), 7.86 (br, 2H), 8.21 (d, 2H, J=6.70).

**6,6-dimethyl-2-(2-aminopyrimidin-4-yl)-1,5,6,7-tetrahydro-4H-pyrrolo[3,2-c]pyridin-4-one hydrochloride**

$^1$H NMR (DMSO-$d_6$/400 MHz): δ ppm 1.3 (sc, *J*=2.7, 0.9, 0.5 Hz, 3 H) 1.5 (sc, *J*=2.7, 0.9, 0.5 Hz, 3 H) 2.5 (sc, *J*=15.6, 1.5, 0.9, 0.5 Hz, 1 H) 2.6 (sc, *J*=15.6, 1.5, 0.9, 0.5 Hz, 1 H) 7.5 (sc, *J*=0.9 Hz, 1 H) 7.7 (sc, *J*=4.8 Hz, 1 H) 8.2 (sc, *J*=4.8 Hz, 1 H) 9.2 (sc,1 H).

**2-(2-phenylamtnopyrimidin-4-yl)-1,5,6,7-tetrahydro-4H-pyrrolo[3,2-c]pyridin-4-one**

1H NMR (400 MHz, Solvent) δ ppm 2.7 (sc, *J*=17,1, 6.2, 5.1, 1.5, 0.9 Hz, 1 H) 2.8 (sc, *J*=17.1, 6.3, 5.1, 1.5, 0.9 Hz, 1 H) 3.5 (sc, *J*=12.2, 6.2, 5.1, 2.5 Hz, 1 H) 3.5 (sc, *J*=12.2, 6.3, 5.1, 2.5 Hz, 1 H) 6.9 (sc, *J*=7.5, 1.2 Hz, 1 H) 7.3 (sc, *J*=8.1, 7.5, 1.6, 0.4 Hz, 1 H) 7.3 (sc, *J*=8.1, 7.5, 1.6, 0.4 Hz, 1 H) 7.6 (sc, *J*=0.9 Hz, 1 H) 7.6 (sc, *J*=8.1, 2.5, 1.2, 0.4 Hz, 1 H) 7.6 (sc. *J*=8.1, 2.5, 1.2, 0.4 Hz, 1 H) 7.7 (sc, *J*=4.8 Hz, 1 H) 8.4 (sc, *J*=4.8 Hz, 1 H).

**2-(2-phenylaminopyrimidin-4-yl)-7-phenyl-1,5,6,7-tetrahydro-4H-pyrrolo[3,2-c]pyridin-4-one hydrochloride**

1 H NMR (400 MHz, Solvent) δ ppm 3.7 (sc, *J*=13.0, 8.0, 4.0 Hz, 1 H) 3.8 (so, *J*=8.0, 6.0, 1.5, 0.9, 0.6 Hz, 1 H) 3.9 (sc, *J*=13.0, 6.0, 3.0 Hz, 1 H) 6.9 (sc, *J*=7.5, 1.2 Hz, 1 H) 7.0 (sc, *J*=7.3, 7.1, 1.4, 1.0 Hz, 1 H) 7.0 (sc, *J*=7.3, 7.1,1.4,1.0 Hz, 1 H) 7.2 (sc, *J*=7.1, 1.2 Hz, 1 H) 7.3 (sc, J=8.1, 7.5, 1.6. 0.4 Hz, 1 H) 7.3 (sc, *J*=8.1, 7.5, 1.6, 0.4 Hz, 1 H)7.4 (sc, *J*=7.3, 1.2, 1.2, 1.0, 0.6 Hz, 1 H) 7.4 (sc, *J*=7.3, 1.2, 1.2, 1.0, 0.6 Hz, 1 H) 7.6 (sc, *J*=0.9 Hz, 1 H) 7.6 (sc, *J*=8.1, 2.5, 1.2, 0.4 Hz, 1 H) 7.6 (sc, *J*=8.1, 2.5, 1.2, 0.4 Hz, 1 H) 7.7 (sc, *J*=4.8 Hz, 1 H) 8.4 (sc, *J*=4.8 Hz, 1 H) 9.2 (sc, 1 H).

**2-(9H-purin-6-yl)-1,5,6,7-tetrahydro-4H-pyrrolo[3,2-c]pyridin-4-one hydrochloride**

$^1$H NMR (DMSO-$d_6$ / 400 MHz) δ ppm 2.91 (t, 2H, J=6.83), 3.42 (t, 2H, J=6.83), 7.16 (br, 1H), 7.77 (s, 1H), 8.59 (s, 1 H), 8.80 (s, 1H), 12.26 (bs, 1 H).

**7-phenyl-2-pyrimidin-4-yl-1,5,6,7-tetrahydro-4H-pyrrolo[3,2-c]pyridin-4-one hydrochloride**

1H NMR (400 MHz, Solvent) δ ppm 3.7 (sc, *J*=13.0, 8.0, 4.0 Hz, 1 H) 3.8 (sc, *J*=8.0, 6.0, 1.5, 0.9, 0.6 Hz, 1 H)

3.9 (sc, J=13.0, 6.0, 3.0 Hz, 1 H) 7.0 (sc, J=7.3, 7.1, 1.4, 1.0 Hz, 1 H) 7.0 (sc, J=7.3, 7.1, 1.4, 1.0 Hz, 1 H) 7.2 (sc, J=7.1, 1.2 Hz, 1 H) 7.4 (sc, J=7.3, 1.2, 1.2, 1.0, 0.6 Hz, 1 H) 7.4 (sc, J=7.3, 1.2, 1.2, 1.0, 0.6 Hz, 1 H) 7.5 (sc, J=0.9 Hz, 1 H) 7.8 (sc, J=4.8, 1.0 Hz, 1 H) 8.9 (sc, J=4.8, 0.5 Hz, 1 H) 9.0 (sc, J=1.0, 0.5 Hz, 1 H) 9.2 (sc, 1 H).

**2-(2-aminopyrimidin-4-yl)-7-phenyl-1,5,6,7-tetrahydro-4H-pyrrolo[3,2-c]pyridin-4-one hydrochloride**

1 H NMR (400 MHz, Solvent) δ ppm 3.7 (sc, J=13.0, 8.0, 4.0 Hz, 1 H) 3.8 (sc, J=8.0, 6.0, 1.5, 0.9, 0.6 Hz, 1 H) 3.9 (sc, J=13.0, 6.0, 3.0 Hz, 1 H) 7.0 (sc, J=7.3, 7.1, 1.4, 1.0 Hz, 1 H) 7.0 (sc, J=7.3, 7.1, 1.4, 1.0 Hz, 1 H) 7.2 (sc, J=7.1, 1.2 Hz, 1 H) 7.4 (sc, J=7.3, 1.2, 1.2, 1.0, 0.6 Hz, 1 H) 7.4 (sc, J=7.3,1.2,1.2.1.0, 0.6 Hz, 1 H) 7.5 (sc, J=0.9 Hz, 1 H) 7.7 (sc, J=4.8 Hz, 1 H) 8.2 (sc, J=4.8 Hz, 1 H) 9.2 (sc, 1 H);

**7-phenyl-2-(9H-purin-6-yl)-1,5,6,7-tetrahydro-4H-pyrrolo[3,2-c]pyridin-4-one hydrochloride**

$^1$H NMR (DMSO-$d_6$/400 MHz) δ ppm 3.93 (dd,2H, J=5.37, 12.80), 4.89 (m, 1H), 7.18 (d, 2H), 7.25 (t, 1 H), 7.32 (t, 2H), 7.85(s, 1H), 8.60 (s, 1 H), 8.78 (s, 1H), 12.27 (bs, 1H).

**2-(2-aminopyrimidin-4-yl)-6-isopropyl-1,5,6,7-tetrahydro-4H-pyrrolo[3,2-c]pyridin-4-one** hydrochloride

1 H NMR (400 MHz, DMSO-D6) δ ppm 0.92 (dd, J=4.15, 6.83 Hz, 6H) 1.86 (m, 1 H) 2.79 (dd, J=9.39, 16.95 Hz, -1H) 2.92 (dd, J=.48, 19.90 Hz, 1H) 3.52 (m, 1H) 7.26 (bs, 1 H) 7.31 (d, J=6.83 Hz, 1 H) 7.52 (bs, 1 H) 8.21 (d, J=6.71 Hz, 1 H) 12.32 (bs, 1 H)

**2-(2-aminopyrimidin-4-yl)-7-methyl-1,5,6,7-tetrahydro-4H-pyrrolo[3,2-c]pyridin-4-one hydrochloride**

1H NMR (400 MHz, DMSO-D6) δ ppm 1.29 (d, J=6.58 Hz, 3 H) 3.06 - 3.24 (m, 2 H) 3.41 - 3.56 (m, 1 H) 7.30 (s, 1 H) 7.35 (d, J=6.83 Hz, 1 H) 7.51 (s, 1 H) 8.03 (s, 2 H) 8.23 (d, J=6.71 Hz, 1 H) 12.24 (s, 1 H)

The racemate, as Boc derivative, was subjected to chiral separation so to obtain the pure enantiomers. Chiral chromatography was performed on CHIRALCELL ® OJ (5 x 50 cm).
Mobile phase was n-Hex/EtOH/MeOH 70:23:7.

Analytical conditions, as hydrochloride: Chiralcell® OJ column, with precolumn, mobile phase n-Hex/EtOH 80: 20.
(7R or 7S)-2-(2-aminopyrimidin-4-yl)-7-methyl-1,5,8,7-tetrahydro-4H-pyrrolo[3,2-c]pyridin-4-one
RT 19.3 min; e.e. 98.7%
(7R or 7S)-2-(2-aminopyrimidin-4-yl)-7-methyl-1,5,6,7-tetrahydro-4H-pyrrolo[3,2-c]pyridin-4-one
RT 24.1 min; e.e. 99.8%
2-(2-aminopyrimidin-4-yl)-6-methyl-1,5,6,7-tetrahydro-4H-pyrrolo[3,2-c]pyridin-4-one hydrochloride
1 H NMR (400 MHz, DMSO-D6) δ ppm 1.24 (d, J=6.34 Hz, 3 H) 2.65 (dd, J=16.71, 10.12 Hz, 1 H) 3.01 (dd, J=16.58, 5.00 Hz, 1 H) 3.73 - 3.85 (m, 1 H) 7.30 (s, 1 H) 7.31 (d, J=7.19 Hz, 1 H) 7.52 (d, J=2.07 Hz, 1 H) 8.03 (s, 2 H) 8.21 (d, J=6.71 Hz, 1 H) 12.34 (s, 1 H)

**2-(2-aminopyrimidin-4-yl)-7,7-dimethyl-1,5,6,7-tetrahydro-4H-pyrrolo[3,2-c]pyridin-4-one hydrochloride**

1H NMR (500 MHz, DMSO-D6) δ ppm 1.37 (s, 6 H) 3.20 (s, 2H) 7.39 - 7.43 (m, 1 H) 7.46 (d, J=6.70 Hz, 1 H) 7.52 (s, 1 H) 8.16 (s, 2 H) 8.28 (d, J=6.70 Hz, 1 H) 12.20 (s, 1 H)

**2-(2-aminopyrimidin-4-yl)-6-isobutyl-1,5,6,7-tetrahydro-4H-pyrrolo[3,2-c]pyridin-4-one hydrochloride**

1 H NMR (400 MHz, DMSO-D6) δ ppm 0.81 - 0.97 (m, 6 H); 1.27 - 1.42 (m, 1 H); 1.42 -1.58 (m, 1 H); 1.64 -1.81 (m, 1 H); 2.70 (dd, J=16.58, 8.41 Hz, 1 H); 3.04 (dd, J=16.58, 5.24 Hz, 1 H); 3.63 - 3.80 (m, 1 H); 7.29 (s, 1H); 7.31 (d, J=6.71 Hz, 1 H); 7.51 (s, 1 H); 8.08 (s, 2 H); 8.21 (d, J=6.71 Hz, 1 H); 12.35 (s, 1 H)

**2-(2-aminopyrimidin-4-yl)-7,7-diethyl-1,5,6,7-tetrahydro-4H-pyrrolo[3,2-c]pyridin-4-one hydrochloride**

1 H NMR (400 MHz, DMSO-D6) δ ppm 0.80(t, J=7.44 Hz, 6 H) 1.73 - 1.85 (m, 4 H) 3.27 (d, J=2.56 Hz, 2 H) 7.31 (s, 1 H) 7.37 - 7.43 (m, 1 H) 7.46 (s, 1 H) 7.91 (s, 2 H) 8.24 (d, J=6.58 Hz, 1 H) 11.98 (s, 1 H)

2-(2-aminopyrimidin-4-yl)-7-isopropyl-1,5,6,7-tetrahydro-4H-pyrrolo[3,2-c]pyridin-4-one

1 H NMR (400 MHz, DMSO-D6) δ ppm 0.91 (t, *J*=6.95 Hz, 6 H) 1.07 (t, *J*=6.95 Hz, 1 H) 1.96 - 2.10 (m, 1 H) 2.66 - 2.74 (m, 1 H) 3.40 - 3.54 (m, 1 H) 6.28 - 6.39 (m, 2 H) 6.95 (d, *J*=5.37 Hz, 1 H) 6.97 (s, 1 H) 7.03 (d, *J*=2.19 Hz, 1 H) 8.16 (d, *J*=5.24 Hz, 1 H) 11.64 (s, 1 H)

## Example 14

2-(2-Amino-pyrimidin-4-yl)-3-iodo-1,5,6,7-tetrahydro-pyrrolo[3,2-c]pyridin-4-one

KOH (61 mg, 1.09 mmol) was added to a solution of 2-(2-amino-pyrimidin-4-yl)-1,5,6,7-tertrahydro-pyrrolo[3,2-c]pyridin-4-one (100 mg, 0.44 mmol) in DMF (5 ml). A solution of iodine (115 mg, 0.45 mmol) in DMF (2 ml) was added. After 30 min the reaction mixture was poured onto ice water (containing 0.5 ml $NH_3$ and 25 mg $K_2S_2O_5$). The yellow precipitate was filtered, washed with cold water and dried. The compound was purified by flash chromatography (DCM-MeOH-30% $NH_4OH$, 95:5:0.5) to give the product as a yellow solid (22 mg, 14% yield). $^1$H NMR (400 MHz, DMSO-d6) δ ppm 2.87 (t, *J*=6.77 Hz, 2H), 3.23 - 3.44 (m, 2 H), 6.41 (s, 2 H), 7.13 (t, *J*=2.68 Hz, 1 H), 7.51 (d, *J*=5.37 Hz, 1 H), 8.29 (d, *J*=5.24 Hz, 1 H), 12.03 (s, 1 H).

## Example 15

2-[2-(Cyclohexylmethyl-amino)-pyrimidin-4-yl]-1,5,6,7-tetrahydro-pyrrolo [3,2-c]pyridin-4-one

2-(2-Amino-pyrimidin-4-yl)-1,5,6,7-tetrahydro-pyrrolo[3,2-c]pyridin-4-one (140 mg, 0.61 mmol), trifluoroacetic acid (565 μl, 7.33 mmol), and cyclohexanecarbaldehyde (151 μl, 1.25 mmol) were mixed in DMF (10 ml). Sodium triacetoxyborohydride (390 mg,1.84 mmol) was added to the above solution and the reaction mixture stirred at room temperature under $N_2$ for 20 h. Additional cyclohexanecarbaldehyde (151 μl, 1.25 mmol) and sodium triacetoxyborohydride (390 mg, 1.84 mmol) were added and the reaction mixture was stirred 20 hours longer. The reaction was quenched with 0.33N NaOH (50 ml), and the product was extracted with DCM (50 ml). The DCM extract was dried ($MgSO_4$), and the solvent was evaporated. The residue was purified by flash chromatography (DCM-MeOH, 95:5) to give the product as a beige solid (80 mg, 40% yield).
$^1$H NMR (400 MHz, DMSO-d6) δ ppm 0.87 - 1.29 (m, 5 H), 1.43 -1.85 (m, 6 H), 2.87 (t, *J*=6.77 Hz, 2 H), 3.18 - 3.31 (m, 2 H), 3.38 - 3.49 (m, 2 H), 6.67 - 6.95 (m, 1 H), 6.85 (d, *J*=5.24 Hz, 1 H), 7.03 (d, *J*=2.19 Hz, 1 H), 7.06 (t, *J*=2.07 Hz, 1 H), 8.15 (d, *J*=5.12 Hz, 1 H), 11.65 (s, 1 H). Analogously the following products can be prepared starting from the corresponding aldehyde:

2-(2-Propylamino-pyrimidin-4-yl)-1,5,6,7-tetrahydro-pyrrolo[3,2-c]pyridin-4-one

$^1$H NMR (400 MHz, DMSO-d6) δ ppm 0.93 (t, *J*=7.38 Hz, 3 H), 1.51-1.64 (m, 2 H), 2.87 (t, *J*=6.89 Hz, 2 H), 3.34 - 3.48 (m, 4 H), 6.81 (s, 1 H), 6.86 (d, *J*=5.24 Hz, 1 H), 7.03 (d, *J*=2.32 Hz, 1 H), 7.05 (t, *J*=2.07 Hz, 1 H), 8.16 (d, *J*=5.24 Hz, 1 H), 11.66 (s, 1 H).

2-(2-Dipropylamino-pyrimidin-4-yl)-1,5,6,7-tetrahydro-pyrrolo[3,2-c]pyridin-4-one

$^1$H NMR (400 MHz, DMSO-d6) δ ppm 0.90 (t, *J*=7.38 Hz, 6 H), 1.53 - 1.70 (m, 4 H), 2.89 (t, J=6.77 Hz, 2 H), 3.38 - 3.49 (m, 2 H), 3.59 (s, 4 H), 6.86 (d, *J*=5.24 Hz, 1 H), 7.05 (d, *J*=2.44 Hz, 1 H), 7.06 - 7.07 (m, 1 H).8.22, (d, *J*=5.12 Hz, 1 H), 11.62 (s, 1 H).

2-(2-Isobutylamino-pyrimidin-4-yl)-1,5,6,7-tetrahydro-pyrrolo[3,2-c]pyridin-4-one

$^1$H NMR (400 MHz, DMSO-d6) δ ppm 0.93 (d, *J*=6.71 Hz, 6 H), 1.81 - 1.95 (m, 1 H), 2.87 (t, *J*=6.83 Hz, 2 H), 3.24 (s, 2 H), 3.41 (td, *J*=6.98, 2.38 Hz, 2 H), 6.71 - 6.94 (m, 1 H), 6.87 (d, *J*=5.24 Hz, 1 H), 7.04 (d, *J*=2.32 Hz, 1 H), 7.06 (t, *J*=2.38 Hz, 1 H), 8.16 (d, *J*=5.12 Hz, 1 H), 11.88 (s, 1 H).

2-{2-[(Furan-2-ylmethyl)-amino]-pyrimidin-4-yl}-1,5,6,7-tetrahydro-pyrrolo[3,2-c]pyridin-4-one

$^1$H NMR (400 MHz, DMSO-d6) δ ppm 2.87 (t, *J*=6.83 Hz, 2 H), 3.19 - 3.48 (m, 2 H), 4.66 (d, *J*=3.66 Hz, 2 H), 6.28 (d, *J*=2.68 Hz, 1 H), 6.38 (dd, *J*=3.17, 1.83 Hz, 1 H), 6.94 (d, *J*=5.24 Hz, 1 H), 7.03 - 7.10 (m, 2 H), 7.28 (s, 1 H), 7.56 (dd, *J*=1.71, 0.85 Hz, 1 H), 8.19 (d, *J*=5.24 Hz, 1 H), 11.75 (s, 1 H).

**N-(4-{[4-(4-Oxo-4,5,6,7-tetrahydro-1H-pyrrolo[3,2-c]pyridin-2-yl)-pyrimidin-2-ylamino]-methyl}-phenyl)acetamide**

$^1$H NMR (400 MHz, DMSO-d6) δ ppm 2.03 (s, 3 H), 2.91 (t, 2 H), 3.38 (m, 2 H), 4.73 (bs, 2 H), 7.1-7.45 (m, 7 H), 7.53 (m, 2 H), 8.17 (d, 1 H), 12.0 (s, 1 H).

**2-(2-Benzylamino-pyrimidin-4-yl)-1,5,6,7-tetrahydro-pyrrolo[3,2-c]pyridin-4-one**

$^1$H NMR (500 MHz, DMSO-d6) δ ppm 2.87 (t, *J*=6.85 Hz, 2 H), 3.42 (td, *J*=6.85, 2.44 Hz, 2 H), 4.68 (s, 2 H), 6.92 (d, *J*=5.18 Hz, 1 H), 7.07 (d, *J*=2.28 Hz, 1 H), 7.08 (t, *J*=2.51 Hz, 1 H), 7.21 - 7.26 (m, 1 H), 7.27 - 7.48 (m, 1 H), 7.32 (t, *J*=7.54 Hz, 2 H), 7.39 (d, *J*=7.76 Hz, 2 H), 8.19 (d, *J*=5.18 Hz, 1 H), 11.73 (s, 1 H).

**Example 16**

**N-[4-(4-oxo-4,5,6,7-tetrahydro-1H-pyrrolo[3,2-c]pyridin-2-yl)pyrimidin-2-yl]benzamide**

To a solution of 2-(2-Benzoylamino-pyrimidin-4-yl)-4-oxo-1,4,6,7-tetrahydro-pyrrolo[3,2-c]pyridine-5-carboxylic acid tert-butyl ester in THF was added HCl 4N In dioxane and the reaction was left under stirring at room temperature for 2h. Solvent evaporation gave the desired product as a solid.
$^1$H-NMR (DMSO-$d_6$) δ ppm 1.45 (2H), 3.44 (2H), 7.22 (1H), 7.36 (1 H), 7.57 (2H,) 7.64 (2H), 8.03 (2H), 8.59 (1H), 11.26 (1H), 12.13 (1H).

**2-(2-Benzoylamino-pyrimidin-4-yl)-4-oxo-1,4,6,7-tetrahydro-pyrrolo[3,2-c]pyridine-5-carboxylic acid tert-butyl ester**

To a mixture of 2-(2-Amino-pyrimidin-4-yl)-4-oxo-1,4,6,7-tetrahydro-pyrrolo[3,2-c]pyridine-5-carboxylic acid tert-butyl ester and $Et_3N$ (4eq.) in dry THF, PhCOCl (2eq.) was added and the mixture was stirred at room temperature under argon overnight. Thus NaOH(1 N) was added; after 30' the solvent was evaporated and the residue was dissolved in water and the aqueous phase was extracted with AcOEt (2X). The organic phase was then washed with aq. $NH_4Cl$ and dried over $Na_2SO_4$. After solvent evaporation the crude product was purified by flash chromatography ($CH_2Cl_2$: MeOH 97:3) to give the pure product as a solid.
$^1$H-NMR (400 MHz, DMSO-$d_6$) δ ppm 1.49 (s, 9H), 2.98 (2H), 3.98 (2H), 7.35 (1H), 7.51-7.66 (m, 4H), 8.00 (2H), 10.82 (1 H), 12.11 (1 H).
Analogously the following products can be prepared starting from the corresponding acylating agent.

**2-methyl-N-[4-(4-oxo-4,5,6,7-tetrahydro-1H-pyrrolo[3,2-c]pyridin-2-yl)pyrimidin-2-yl]propanamide hydrochloride**

$^1$H-NMR (400 MHz, DMSO-$d_6$): δ ppm 1.15 (6H, d), 2.93 (3H, m), 3.43 (2H), 7.25 (1 H), 7.36 (1H, s) 7.58 (1 H), 8.51 (1H), 11.03 (1 H), 12.16 (1 H).

**N-[4-(4-oxo-4,5,6,7-tetrahydro-1H-pyrrolo[3,2-c]pyridin-2-yl)pyrimidin-2-yl]thlophene-2-carboxamide hydrochloride**

$^1$H-NMR (400 MHz, DMSO-$d_6$): δ ppm 2.91 (2H), 3.44 (2H), 7.22 (1 H), 7.27 (1H), 7.36 (1H,) 7.63 (1H), 7.97 (1H), 8.22 (1H), 8.59 (1H), 11.33 (1 H), 12.10 (1H).

**N-[4-(4-oxo-4,5,6,7-tetrahydro-1H-pyrrolo[3,2-c]pyridin-2-yl)pyrimidin-2-yl]-2-phenylacetamide hydrochloride**

$^1$H-NMR (400 MHz, DMSO-$d_6$): δ ppm 2.90 (2H), 3.42 (2H), 3.93(2H, s), 7.21 (1H), 7.31 (5H) 7.34 (1H), 7.54 (1H), 8.51 (1 H), 11.03 (1H), 12.06 (1H),

**N-[4-(4-oxo-4,5,6,7-tetrahydro-1H-pyrrolo[3,2-c]pyridin-2-yl)pyrimidin-2-yl]acetamide hydrochloride**

$^1$H-NMR (400 MHz, DMSO-$d_6$): δ ppm 2.31 (3H, s), 2.92 (2H, t), 3.51 (2H), 7.26 (1 H), 7.37 (1H, s) 7.60 (1 H), 8.50 (1 H), 11.11 (1H), 12.22 (1 H).

**ethyl 4-(4-oxo-4,5,6,7-tetrahydro-1H-pyrrrolo[3,2-c]pyridin-2-yl)pyrimidin-2-ylcarbamate**

$^1$H NMR (400 MHz, DMSO-d6) δ ppm 1.26 (t, 3H), 2.88 (t, 2H), 3.2 - 3.5 (m, 2 H), 4.17 (q, 2H), 7.12 (t, 1 H), 7.2 (d, 1 H), 7.4 (d, 1 H), 8.48 (d, 1 H), 10.05 (s, 1 H), 11.80 (s, 1 H).

## Claims

1. A compound of formula (I) for treating cell proliferative disorders caused by and/or associated with an altered protein kinase activity selected from the group consisting of cancer, Alzheimer's disease, viral infections, auto-immune diseases, neurodegenerative disorders, benign prostate hyperplasia, familial adenomatosis, polyposis, neuro-fibromatosis, psoriasis, vascular smooth cell proliferation associated with atherosclerosis, pulmonary fibrosis, arthritis, glomerulonephritis and post-surgical stenosis and restenosis, wherein the compound is to be administered in an effective amount to a mammal in need thereof,

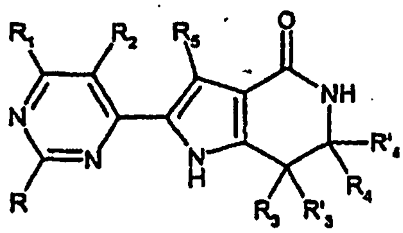

(I)

wherein
R is a hydrogen atom or a group selected from amino, arylamino, optionally substituted $C_1$-$C_6$ alkylamino, optionally substituted arylalkylamino, heteroarylalkylamino, $C_1$-$C_6$ dialkylamino and acylamino;
$R_1$ and $R_2$ are, each independently, a hydrogen or halogen atom, a straight or branched $C_1$-$C_6$ alkyl group, an amino or arylamino group or, taken together with the pyrimidine bond to which they are linked, $R_1$ and $R_2$ may form a divalent -NH-CH=N-, -N=CH-NH- or -NH-CH=CH- group;
$R_3$, $R'_3$, $R_4$ and $R'_4$ are, each independently, a hydrogen atom or a group selected from straight or branched $C_1$-$C_6$ alkyl, $C_3$-$C_6$ cycloalkyl, heterocyclyl, aryl, cycloalkyl-alkyl, heterocyclyl-alkyl or aryl-alkyl; or $R_3$ and $R'_3$ or $R_4$ and $R'_4$, taken together, form a $C_3$-$C_6$ cyclic alkyl group;
$R_5$ is a hydrogen or halogen atom or a straight or branched $C_1$-$C_6$ alkyl group and pharmaceutical acceptable salts thereof.

2. The compound according to claim 1 wherein the cancer is selected from the group consisting of carcinoma, squamous cell carcinoma, hematopoietic tumors of myeloid or lymphoid lineage, tumors of mesenchymal origin, tumors of the central and peripheral nervous system, melanoma, seminoma, teratocarcinoma, osteosarcoma, xeroderma pigmentosum, keratoxanthoma, thyroid follicular cancer, and Kaposi's sarcoma.

3. The compound according to claim 1 wherein the mammal in need thereof is further to be subjected to a radiation therapy or chemotherapy regimen in combination with at least one cytostatic or cytotoxic agent.

4. The compound according to claim 1 wherein the mammal in need thereof is a human.

5. A compound of formula (I)

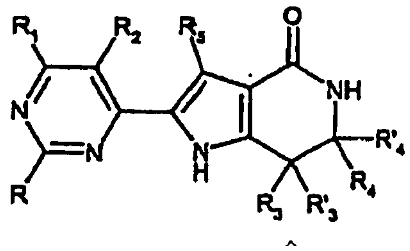

(I)

wherein
R is a hydrogen atom or a group selected from amino, arylamino, optionally substituted $C_1$-$C_6$ alkylamino, optionally substituted arylalkylamino, heteroarylalkylamino, $C_1$-$C_6$ dialkylamino and acylamino;
$R_1$ and $R_2$ are, each independently, a hydrogen or halogen atom, a straight or branched $C_1$-$C_6$ alkyl group, an amino or arylamino group or, taken together with the pyrimidine bond to which they are linked, $R_1$ and $R_2$ may form a divalent -NH-CH=N-, -N=CH-NH- or -NH-CH=CH- group;
$R_3$, $R'_3$, $R_4$ and $R'_4$ are, each independently, a hydrogen atom or a group selected from straight or branched $C_1$-$C_6$ alkyl, $C_3$-$C_6$ cycloalkyl, heterocyclyl, aryl, cycloalkyl-alkyl, heterocyclyl-alkyl or aryl-alkyl; or $R_3$ and $R'_3$ or $R_4$ and $R'_4$, taken together, form a $C_3$-$C_6$ cyclic alkyl group; $R_5$ is a hydrogen or halogen atom or a straight or branched $C_1$-$C_6$ alkyl group and pharmaceutical acceptable salts thereof, provided that the compound 2-[2-(dimethylamino)-5-fluoropyrimidin-4-yl]-1,5,6,7-tetrahydro-4H-pyrrolo[3,2-c]pyridin-4-one is excluded.

6. A compound of formula (I) according to claim 5 wherein R is hydrogen, amino or phenylamino; $R_1$ and $R_2$ are both hydrogen atoms or, taken together with the pyrimidine bond to which they are linked, form a divalent -NH-CH=N- group; and $R_3$,-$R'_3$, $R_4$, $R'_4$ and $R_5$ are as above defined.

7. A compound of formula (I) according to claim 5 wherein $R_3$ and $R'_3$ are both hydrogen atoms or one of them is a phenyl group and the remaining one is a hydrogen atom; and R, $R_1$, $R_2$, $R_4$, $R'_4$ and $R_5$ are as above defined.

8. A compound of formula (I) according to claim 5 wherein $R_4$ and $R'_4$ are both hydrogen atoms or both methyl groups; and R, $R_1$, $R_2$, $R_3$, $R'_3$, $R_4$, $R'_4$ and $R_5$ are as above defined.

9. A compound of formula (I) according to claim 5 wherein $R_5$ is a hydrogen atom and R, $R_1$, $R_2$, $R_3$, $R'_3$, $R_4$ and $R'_4$ are as above defined.

10. A compound of formula (I) according to claim 5, optionally in the form of a pharmaceutical acceptable salt thereof, selected from the group consisting of:

2-(2-aminopyrimidin-4-yl)-1,5,6,7-tetrahydro-4H-pyrrolo[3,2-c]pyridin-4-one hydrochloride;
2-(2-aminopyrimidin-4-yl)-7-phenyl-1,5,6,7-tetrahydro-4H-pyrrolo[3,2-c]pyridin-4-one hydrochloride;
2-(2-phenylaminopyrimidin-4-yl)-1,5,6,7-tetrahydro-4H-pyrrolo[3,2-c] pyridin-4-one hydrochloride;
2-(9H-purin-6-yl)-1,5,6,7-tetrahydro-4H-pyrrolo[3,2-c]pyridin-4-one hydrochloride;
2-pyrimidin-4-yl-1,5,6,7-tetrahydro-4H-pyrrolo[3, 2-c]pyridin-4-one hydrochloride;
7-phenyl-2-(9H-purin-6-yl)-1,5,6,7-tetrahydro-4H-pyrrolo[3,2-c] pyridin-4-one hydrochloride;
2-(2-aminopyrimidin-4-yl)-6,6-dimethyl-1,5.,6,7-tetrahydro-4H-pyrrolo[3, 2-c]pyridin-4-one hydrochloride;
7-phenyl-2-pyrimidin-4-yl-1,5,6,7-tetrahydro-4H-pyrrolo[3,2-c]pyridin-4-one hydrochloride;
2-(2-anilinopyrimidin-4-yl)-7-phenyl-1,5,6,7-tetrahydro-4H-pyrrolo[3,2-c]pyridin-4-one hydrochloride;
2-(2-arninopyrimidin-4-yl)-6-isopropyl-1,5,6,7-tetrahydro-4H-pyrrolo[3,2-c]pyridin-4-one;
2-(2-aminopyrimidin-4-yl)-7-methyl-1,5,6,7-tetrahydro-4H-pyrrolo[3,2-c]pyridin-4-one;
2-(2-aminopyrimidin-4-yl)-6-methyl-1,5,6,7-tetrahydro-4H-pyrrolo[3,2-c]pyridin-4-one;
2-(2-aminopyrimidin-4-yl)-7,7-dimethyl-1,5,6,7-tetrahydro-4H-pyrrolo[3,2-c]pyridin-4-one;
2-(2-aminopyrimidin-4-yl)-6-isobutyl-1,5,6,7-tetrahydro-4H=pyrrolo[3,2-c]pyridin-4-one;
ethyl 4-(4-oxo-4,5.,6,7-tetrahydro-1H-pyrrolo[3,2-c]pyridin-2-yl)pyrimidin-2-ylcarbamate;
(7R or 7S)-2-(2-aminopyrimidin-4-yl)-7-methyl-1,5,6,7-tetrahydro-4H-pyrrolo[3,2-c]pyridin-4-one;
(7R or 7S)-2-(2-aminopyrimidin-4-yl)-7-methyl-1,5,6,7-tetrahydro-4H-pyrrolo[3,2-c]pyridin-4-one;
2-(2-aminopyrimidin-4-yl)-3-iodo-1,5,6,7-tetrahydro-4H-pyrrolo[3,2-c]pyridin-4-one;
2-(2-aminopyrimidin-4-yl)-7,7-diethyl-1,5,6,7-tetrahydro-4H-pyrrolo[3,2-c]pyridin-4-one;
2-{2-[(2-furylmethyl)amino]pyrimidin-4-yl}-1,5,6,7-tetrahydro-4H-pyrrolo[3,2-c]pyridin-4-one;

N-[4-(4-oxo-4,5,6,7-tetrahydro-1H-pyrrolo[3,2-c]pyridin-2-yl)pyrimidin-2-yl]benzamide;
2-(2-aminopyrimidin-4-yl)-7-isopropyl-1,5,6,7-tetrahydro-4H-pyrrolo[3, 2-c]pyridin-4-one;
2-[2-(benzylamino)pyrimidin-4-yl]-1,5,6,7-tetrahydro-4H-pyrrolo[3,2-c]pyridin-4-one;
2-[2-(propylamino)pyrimidin-4-yl]-1,5,6,7-tetrahydro-4H-pyrrolo[3,2-c]pyridin-4-one;
2-[2-(isobutylamino)pyrimidin-4-yl]-1,5,6,7-tetrahydro-4H-pyrrolo[3,2-c]pyridin-4-one;
2-{2-[(cyclohexylmethyl)amino]pyrimidin-4-yl}-1,5,6,7-tetrahydro-4H-pyrrolo[3,2-c]pyridin-4-one;
2-{2-[(2-furylmethyl)amino]pyrimidin-4-yl}-1,5,6,7-tetrahydro-4H-pyrrolo[3,2-c]pyridin-4-one and
N-[4-({[4-(4-oxo-4,5,6,7-tetrahydro-1H-pyrrolo[3,2-c]pyridin-2-yl)pyrimidin-2-yl]amino}methyl)phenyl]acetamide trifluoroacetate.

**11.** A process for preparing the compounds of formula (I) and the pharmaceutically acceptable salts thereof, according to claim 5, which process comprises:

a) halogenating a compound of formula (II) so as to obtain a compound of formula (III)

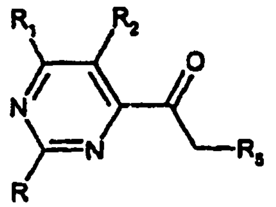

(II)

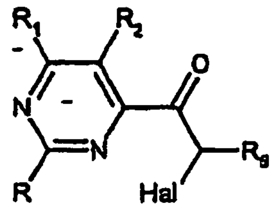

(III)

wherein R, $R_1$ and $R_2$ have the above reported meanings, $R_5$ is a hydrogen atom or a straight or branched $C_1$-$C_6$ alkyl group and Hal represents a suitable halogen atom, preferably bromine or chlorine;
b) reacting the compound of formula (III) with a compound of formula (IV)

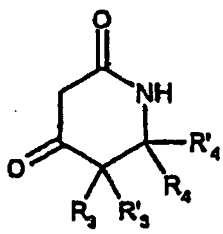

(IV)

wherein $R_3$, $R'_3$, $R_4$ and $R'_4$ have the above reported meanings, so as to obtain a compound of formula (I) and, optionally, converting it into another compound of formula (I) and/or into a pharmaceutically acceptable salt thereof.

**12.** The process according to claim 11 wherein step (a) is carried out by brominating or chlorinating the compound of formula (II).

**13.** The process according to claim 12 wherein, within the compound of formula (III), Hal represents a bromine or chlorine atom.

**14.** A pharmaceutical composition comprising a therapeutically effective amount of a compound of formula (I) or a pharmaceutically acceptable salt thereof, as defined in claim 5 except that the compound 2-[2-(dimethylamino)-5-fluoropyrimidin-4-yl]-1,5,6,7-tetrahydro-4H-pyrrolo[3,2-c]pyridin-4-one is not excluded, and at least one pharmaceutically acceptable excipient, carrier and/or diluent.

**15.** A pharmaceutical composition according to claim 14 further comprising one or more chemotherapeutic agents.

**16.** A product or kit comprising a compound of formula (I) or a pharmaceutically acceptable salt thereof, as defined in

claim 5 except that the compound 2-[2-(dimethylamino)-5-fluoropyrimidin-4-yl]-1,5,6,7-tetrahydro-4H-pyrrolo[3,2-c]pyridin-4-one is not excluded, or pharmaceutical compositions thereof as defined in claim 14, and one or more chemotherapeutic agent, as a combined preparation for simultaneous, separate or sequential use in anticancer therapy.

**17.** A compound of formula (I) or a pharmaceutically acceptable salt thereof, as defined in claim 5 except that the compound 2-[2-(dimethylamino)-5-fluoropyrimidin-4-yl]-1,5,6,7-tetrahydro-4H-pyrrolo[3,2-c]pyridin-4-one is not excluded, for use as a medicament.

**18.** Use of a compound of formula (I) or a pharmaceutically acceptable salt thereof, as defined in claim 5 except that the compound 2-[2-(dimethylamino)-5-fluoropyrimidin-4-yl]-1,5,6,7-tetrahydro-4H-pyrrolo[3,2-c]pyridin-4-one is not excluded, in the manufacture of a medicament with antitumor activity.

## Patentansprüche

**1.** Eine Verbindung der Formel (I) zur Behandlung von zellproliferativen Erkrankungen, die bewirkt sind durch und/oder assoziiert sind mit einer veränderten Proteinkinaseaktivität, gewählt aus der Gruppe bestehend aus Krebs, Alzheimer'scher Krankheit, viralen Infektionen, auto-Irnrnunerkrankungen, neurodegenerativen Erkrankungen, benigner Prostatahyperplasie, familiärer Adenomatosis, Polyposis, Neuro-Fibromatosis, Psoriasis, glatter Gefäßzellproliferation assoziiert mit Atherosklerose, Lungenfibrose, Arthritis, Glomerulonephritis und post-operativer Stenose und Restenose, worin die Verbindung in einer wirksamen Menge an ein Säugetier, das dies benötigt, verabreicht werden soll,

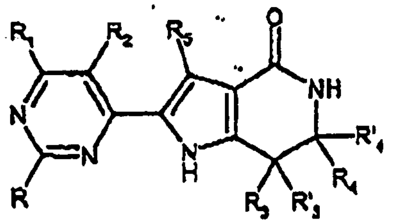

(I)

worin

R ein Wasserstoffatom ist oder eine Gruppe gewählt aus Amino, Arylamino, wahlweise substituiertem $C_1$-$C_6$ Alkylamino, wahlweise substituiertem Arylalkylamino, Heteroarylalkylamino, $C_1$-$C_6$ Dialkylamino und Acylamino; $R_1$ und $R_2$ sind, jeweils unabhängig, ein Wasserstoff oder Halogenatom, eine gerade oder verzweigte $C_1$-$C_6$ Alkylgruppe, eine Amino oder Arylaminogruppe oder zusammengenommen mit der Pyrimidinbindung, an welche sie angeknüpft sind, können $R_1$ und $R_2$ eine divalente -NH-CH=N-, -N=CH-NH- oder -NH-CH=CH-Gruppe bilden; $R_3$, $R'_3$, $R_4$ und $R'_4$ sind, jeweils unabhängig, ein Wasserstoffatom oder eine Gruppe gewählt aus geradem oder verzweigtem $C_1$-$C_6$ Alkyl, $C_3$-$C_6$ Cycloalkyl, Heterocyclyl, Aryl, Cycloalkyl-Alkyl, Heterocyclyl-Alkyl oder Aryl-Alkyl; oder $R_3$ und $R'_3$ oder $R_4$ und $R'_4$, zusammengenommen, bilden eine $C_3$-$C_6$ cyclische Alkylgruppe; $R_5$ ist ein Wasserstoff oder Halogenatom oder eine gerade oder verzweigte $C_1$-$C_6$ Alkylgruppe und pharmazeutisch verträgliche Salze davon.

**2.** Die Verbindung gemäß Anspruch 1, worin der Krebs gewählt ist aus der Gruppe bestehend aus Karzinom, Plattenepitelkarzinom, hämatopoietischen Tumoren myeloider oder lymphoider Abstammung, Tumore mesenchymalen Ursprungs, Tumore des zentralen und peripheren Nervensystems, Melanom, Seminom, Teratokarzinom, Osteosarkom, Xeroderma Pigmentosum, Keratoxanthom, Schilddrüsenfollikelkrebs, und Kaposi Sarkom.

**3.** Die Verbindung gemäß Anspruch 1, worin das Säugetier, das dies benötigt, weiter einer Bestrahlungstherapie oder einem Chemotherapieregime in Kombination mit mindestens einem cytostatischen oder cytotoxischen Wirkstoff unterzogen werden soll.

**4.** Die Verbindung gemäß Anspruch 1, worin das Säugetier, das dies benötigt ein Mensch ist.

5. Eine Verbindung der Formel (I)

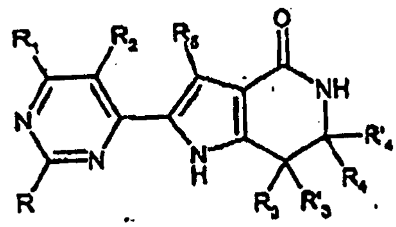

(I)

worin
R eine Wasserstoffatom oder eine Gruppe gewählt aus Amino, Arylamino, wahlweise substituiertem $C_1$-$C_6$ Alkylamino, wahlweise substituiertem Arylalkylamino, Heteroarylalkylamino, $C_1$-$C_6$ Dialkylamino und Acylamino ist; $R_1$ und $R_2$ sind jeweils unabhängig ein Wasserstoff oder Halogenatom, eine gerade oder verzweigte $C_1$-$C_6$ Alkylgruppe, eine Amino oder Arylaminogruppe oder, zusammengenommen mit der Pyrimidinbindung, an welche sie angekünpft sind, können $R_1$ und $R_2$ eine divalente -NH-CH=N-, -N=CH-NH- oder -NH-CH=CH-Gruppe bilden; $R_3$, $R'_3$, $R_4$ und $R'_4$ sind, jeweils unabhängig, ein Wasserstoffatom oder eine Gruppe gewählt aus geradem oder verzweigtem $C_1$-$C_6$ Alkyl, $C_3$-$C_6$ Cycloalkyl, Heterocyclyl, Aryl, Cycloalkyl-Alkyl, Heterocyclyl-Alkyl oder Aryl-Alkyl; oder $R_3$ und $R'_3$ oder $R_4$ und $R'_4$, zusammengenommen, bilden eine $C_3$-$C_6$ cyclische Alkylgruppe; $R_5$ ist ein Wasserstoff oder Halogenatom oder eine gerade oder verzweigte $C_1$-$C_6$ Alkylgruppe und pharmazeutisch verträgliche Salze davon, vorausgesetzt, dass die Verbindung 2-[2-(Dimethylamino)-5-fluorpyrimidin-4-yl]-1,5,6,7-tetrahydro-4H-pyrrolo[3,2-c]pyridin-4-on ausgeschlossen ist.

6. Eine Verbindung der Formel (I) gemäß Anspruch 5, worin R Wasserstoff, Amino oder Phenylamino ist; $R_1$ und $R_2$ sind beides Wasserstoffatome oder bilden, zusammengenommen mit der Pyrimidinbindung mit welcher sie verknüpft sind, eine divalente -NH-CH=N-Gruppe; und $R_3$, $R'_3$, $R_4$, $R'_4$ und $R_5$ sind wie oben definiert.

7. Eine Verbindung der Formel (I) gemäß Anspruch 5, worin $R_3$ und $R'_3$ beides Wasserstoffatome sind, oder eines davon ist eine Phenylgruppe und das andere ist ein Wasserstoffatom; und R, $R_1$, $R_2$, $R_4$, $R'_4$ und $R_5$ sind wie oben definiert.

8. Eine Verbindung der Formel (I) gemäß Anspruch 5, worin $R_4$ und $R'_4$ beides Wasserstoffatome oder beides Methylgruppen sind; und R, $R_1$, $R_2$, $R_3$, $R'_3$, $R_4$, $R'_4$ und $R_5$ sind wie oben definiert.

9. Eine Verbindung der Formel (I) gemäß Anspruch 5, worin $R_5$ ein Wasserstoffatom ist und R, $R_1$, $R_2$, $R_3$, $R'_3$, $R_4$, und $R'_4$ sind wie oben definiert.

10. Eine Verbindung der Formel (I) gemäß Anspruch 5, wahlweise in der Form eines pharmazeutisch verträglichen Salzes davon, gewählt aus der Gruppe bestehend aus:

2-(2-Aminopyrimidin-4-yl)-1,5,6,7-tetrahydro-4H-pyrrolo[3,2-c] pyridin-4-on Hydrochlorid;
2-(2-Aminopyrimidin-4-yl)-7-phenyl-1,5,6,7-tetrahydro-4H-pyrrolo [3,2-c]pyridin-4-on Hydrochlorid;
2-(2-Phenylaminopyrimidin-4-yl)-1,5,6,7-tetrahydro-4H-pyr-rolo [3,2-c]pyridin-4-on Hydrochlorid;
2-(9H-Purin-6-yl)-1,5,6,7-tetrahydro-4H-pyrrolo[3,2-c]pyridin-4-on Hydrochlorid;
2-Pyrimidin-4-yl-1,5,6,7-tetrahydro-4H-pyrrolo[3,2-c]pyridin-4-on Hydrochlorid;
7-Phenyl-2-(9H-purin-6-yl)-1,5,6,7-tetrahydro-4H-pyrrolo[3,2-c] pyridin-4-on Hydrochlorid;
2-(2-Aminopyrimidin-4-yl)-6,6-dimethyl-1,5,6,7-tetrahydro-4H-pyrrolo[3,2-c]pyridin-4-on Hydrochlorid;
7-Phenyl-2-pyrimidin-4-yl-1,5,6,7-tetrahydro-4H-pyrrolo[3,2-c] pyridin-4-on Hydrochlorid;
2-(2-Anilinopyrimidin-4-yl)-7-phenyl-1,5,6,7-tetrahydro-4H-pyrrolo[3,2-c]pyridin-4-on Hydrochlorid;
2-(2-Aminopyrimidin-4-yl)-6-isopropyl-1,5,6,7-tetrahydro-4H-pyrrolo[3,2-c]pyridin-4-on;
2-(2-Aminopyrimidin-4-yl)-7-methyl-1,5,6,7-tetrahydro-4H-pyrrolo [3,2-c]pyridin-4-on;
2-(2-Aminopyrimidin-4-yl)-6-methyl-1,5,6,7-tetrahydro-4H-pyrrolo [3,2-c]pyridin-4-on;
2-(2-Aminopyrimidin-4-yl)-7,7-dimethyl-1,5,6,7-tetrahydro-4H-pyrrolo[3,2-c]pyridin-4-on;
2-(2-Aminopyrimidin-4-yl)-6-isobutyl-1,5,6,7-tetrahydro-4H-pyrrolo[3,2-c]pyridin-4-on;
Ethyl 4-(4-Oxo-4,5,6,7-tetrahydro-1H-pyrrolo[3,2-c]pyridin-2-yl) pyrimidin-2-ylcarbamat;
(7R oder 7S)-2-(2-Aminopyrimidin-4-yl)-7-methyl-1,5,6,7-tetrahydro-4H-pyrrolo[3,2-c]pyridin-4-on;

(7R oder 7S)-2-(2-Aminopyrimidin-4-yl)-7-methyl-1,5,6,7-tetrahydro-4H-pyrrolo[3,2-c]pyridin-4-on;
2-(2-Aminopyrimidin-4-yl)-3-jod-1,5,6,7-tetrahydro-4H-pyrrolo [3,2-c]pyridin-4-on;
2-(2-Aminopyrimidin-4-yl)-7,7-diethyl-1,5,6,7-tetrahydro-4H-pyrrolo[3,2-c]pyridin-4-on;
2-{2-[(2-Furylmethyl)amino]pyrimidin-4-yl}-1,5,6,7-tetrahydro-4H-pyrrolo[3,2-c]pyridin-4-on;
N-[4-(4-Oxo-4,5,6,7-tetrahydro-1H-pyrrolo[3,2-c]pyridin-2-yl) pyrimidin-2-yl]benzamid;
2-(2-Aminopyrimidin-4-yl)-7-isopropyl-1,5,6,7-tetrahydro-4H-pyrrolo[3,2-c]pyridin-4-on;
2-[2-(Benzylamino)pyrimidin-4-yl]-1,5,6,7-tetrahydro-4H-pyrrolo [3,2-c]pyridin-4-on;
2-[2-(Propylamino)pyrimidin-4-yl]-1,5,6,7-tetrahydro-4H-pyrrolo [3,2-c]pyridin-4-on;
2-[2-(Isobutylamino)pyrimidin-4-yl]-1,5,6,7-tetrahydro-4H-pyrrolo[3,2-c]pyridin-4-on;
2-{2-[(Cyclohexylmethyl)amino]pyrimidin-4-yl}-1,5,6,7-tetrahydro-4H-pyrrolo[3,2-c]pyridin-4-on;
2-{2-[(2-Furylmethyl)amino]pyrimidin-4-yl}-1,5,6,7-tetrahydro-4H-pyrrolo[3,2-c]pyridin-4-on und
N-[4-({[4-(4-Oxo-4,5,6,7-tetrahydro-1H-pyrrolo[3,2-c]pyridiri-2-yl)pyrimidin-2-yl]amino}methyl)phenyl]acetamid Trifluoracetat.

**11.** Ein Verfahren zur Herstellung der Verbindungen der Formel (I) und der pharmazeutisch verträglichen Salze davon, gemäß Anspruch 5, wobei das Verfahren folgendes umfasst:

a) das Halogenieren einer Verbindung der Formel (II) um eine Verbindung der Formel (III) zu erhalten

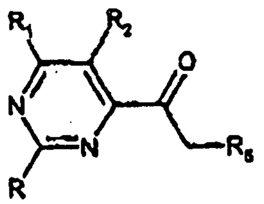
(II)

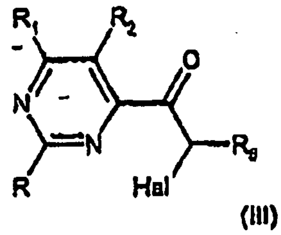
(III)

worin R, $R_1$, und $R_2$ die oben angegebenen Bedeutungen haben, $R_5$ ist ein Wasserstoffatom oder eine gerade oder verzweigte $C_1$-$C_6$ Alkylgruppe und Hal stellt ein geeignetes Halogenatom dar, vorzugsweise Brom oder Chlor;
b) Reagieren der Verbindung der Formel (III) mit einer Verbindung der Formel (IV)

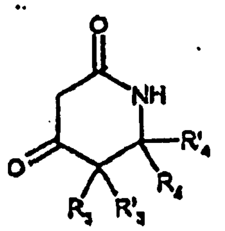
(IV)

worin $R_3$, $R'_3$, $R_4$ und $R'_4$ die oben angegebenen Bedeutungen haben, um eine Verbindung der Formel (I) zu erhalten, und wahlweise Umwandeln dieser in eine andere Verbindung der Formel (I) und/oder in ein pharmazeutisch verträgliches Salz davon.

**12.** Das Verfahren gemäß Anspruch 11, worin Schritt (a) durch Bromieren oder Chlorieren der Verbindung von Formel (II) ausgeführt wird.

**13.** Das Verfahren gemäß Anspruch 12, worin, innerhalb der Verbindung der Formel (III) Hal ein Brom- oder Chloratom darstellt.

**14.** Eine pharmazeutische Zusammensetzung, die eine therapeutisch wirksame Menge einer Verbindung der Formel (I) oder ein pharmazeutisch verträgliches Salz davon wie in Anspruch 5 definiert umfasst, außer dass die Verbindung

2-[2-(Dimethylamino)-5-fluorpyrimidin-4-yl]-1,5,6,7-tetrahydro-4H-pyrrolo[3,2-c]pyridin-4-on nicht ausgeschlossen ist, und mindestens ein pharmazeutisch verträgliches Bindemittel, Träger und/oder Verdünnungsmittel.

**15.** Eine pharmazeutische Zusammensetzung gemäß Anspruch 14, die weiterhin einen oder mehrere chemotherapeutische Wirkstoffe umfasst.

**16.** Ein Produkt oder ein Kit, der eine Verbindung der Formel (I) oder ein pharmazeutisch verträgliches Salz davon, wie in Anspruch 5 definiert umfasst, außer dass die Verbindung 2-[2-(Dimethylamino)-5-fluorpyrimidin-4-yl]-1,5,6,7-tetrahydro-4H-pyrrolo[3,2-c]pyridin-4-on nicht ausgeschlossen ist, oder pharmazeutische Zusammensetzungen davon, wie in Anspruch 14 definiert, und einem oder mehrere chemotherapeutische Wirkstoffe, als eine kombinierte Zubereitung für die gleichzeitige, separate oder aufeinanderfolgende Verwendung in der Antikrebstherapie.

**17.** Eine Verbindung der Formel (I) oder ein pharmazeutisch verträgliches Salz davon, wie in Anspruch 5 definiert, außer dass die Verbindung 2-[2-(Dimethylamino)-5-fluorpyrimidin-4-yl]-1,5,6,7-tetrahydro-4H-pyrrolo[3,2-c]pyridin-4-on nicht ausgeschlossen ist, für die Verwendung als ein Medikament.

**18.** Verwendung einer Verbindung der Formel (I) oder eines pharmazeutisch verträglichen Salzes davon, wie in Anspruch 5 definiert, außer dass die Verbindung 2-[2-(Dimethylamino)-5-fluorpyrimidin-4-yl]-1,5,6,7-tetrahydro-4H-pyrrolo [3,2-c] pyridin-4-on nicht ausgeschlossen ist, in der Herstellung eines Medikaments mit Antitumorwirksamkeit.

**Revendications**

**1.** Composé de formule (I) destiné au traitement de troubles de la prolifération cellulaire provoqués par, et/ou associés à, une activité de protéine-kinase modifiée choisie dans le groupe consistant en le cancer, la maladie d'Alzheimer, des infections virales, des maladies auto-immunes, des troubles neurodégénératifs, l'hyperplasie prostatique bénigne, l'adénomatose familiale, la polypose, la neurofibromatose, le psoriasis, la prolifération des cellules du tissu lisse associée à l'athérosclérose, la fibrose pulmonaire, l'arthrite, la glomérulonéphrite et la sténose et la resténose postchirurgicales, le composé étant destiné à être administré en une quantité efficace à un mammifère en ayant besoin,

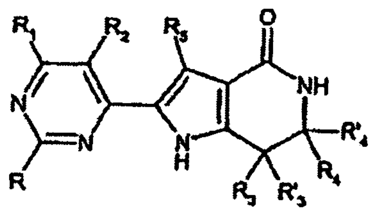

(I)

formule dans laquelle
R représente un atome d'hydrogène ou un groupe choisi entre des groupes amino, arylamino, alkylamino en $C_1$ à $C_6$ facultativement substitué, arylalkylamino facultativement substitué, hétéroarylalkylamino, di(alkyle en $C_1$ à $C_6$) amino et acylamino ;
$R_1$ et $R_2$ représentent chacun indépendamment un atome d'hydrogène ou d'halogène, un groupe alkyle en $C_1$ à $C_6$ droit ou ramifié, un groupe amino ou arylamino ou bien, pris conjointement avec le noyau pyrimidine auquel ils sont liés, $R_1$ et $R_2$ peuvent former un groupe -NH-CH=N-, -N=CH-NH- ou -NH-CH=CH- ; $R_3$, $R'_3$, $R_4$ et $R'_4$ représentent chacun indépendamment un atome d'hydrogène ou un groupe choisi entre des groupes alkyle en $C_1$ à $C_6$ droit ou ramifié, cycloalkyle en $C_3$ à $C_6$, hétérocyclyle, aryle, cycloalkylalkyle, hétérocyclylalkyle et arylalkyle ; ou bien $R_3$ et $R'_3$ ou $R_4$ et $R'_4$, pris conjointement, forment un groupe alkyle cyclique en $C_3$ à $C_6$ ;
$R_5$ représente un atome d'hydrogène ou d'halogène ou un groupe alkyle en $C_1$ à $C_6$ droit ou ramifié ; et de ses sels pharmaceutiquement acceptables.

**2.** Composé suivant la revendication 1, le cancer étant choisi dans le groupe consistant en un carcinome, un carcinome à cellules squameuses, des tumeurs hématopoïétiques de la lignée myéloide ou lymphoïde, des tumeurs d'origine mésenchymateuse, des tumeurs du système nerveux central et du système nerveux périphérique, un mélanome, un séminome, un tératocarcinome, un ostéosarcome, le xeroderma pigmentosum, un kératoxanthome, le cancer folliculaire de la thyroïde et le sarcome de Kaposi.

**3.** Composé suivant la revendication 1, le mammifère en ayant besoin étant en outre destiné à être soumis à un schéma de radiothérapie ou de chimiothérapie en association avec au moins un agent cytostatique ou cytotoxique.

**4.** Composé suivant la revendication 1, le mammifère en ayant besoin étant un être humain.

**5.** Composé de formule (I)

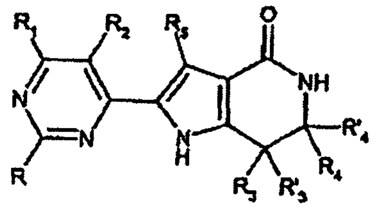

(I)

dans laquelle
R représente un atome d'hydrogène ou un groupe choisi entre des groupes amino, arylamino, alkylamino en $C_1$ à $C_6$ facultativement substitué, arylalkylamino facultativement substitué, hétéroarylalkylamino, di(alkyle en $C_1$ à $C_6$) amino et acylamino ;
$R_1$ et $R_2$ représentent chacun indépendamment un atome d'hydrogène ou d'halogène, un groupe alkyle en $C_1$ à $C_6$ droit ou ramifié, un groupe amino ou arylamino ou bien, pris conjointement avec le noyau pyrimidine auquel ils sont liés, $R_1$ et $R_2$ peuvent former un groupe divalent -NH-CH=N-, -N=CH-NH- ou -NH-CH=CH- ; $R_3$, $R'_3$, $R_4$ et $R'_4$ représentent chacun indépendamment un atome d'hydrogène ou un groupe choisi entre des groupes alkyle en $C_1$ à $C_6$ droit ou ramifié, cycloalkyle en $C_3$ à $C_6$, hétérocyclyle, aryle, cycloalkylalkyle, hétérocyclylalkyle et arylalkyle ; ou bien $R_3$ et $R'_3$ ou $R_4$ et $R'_4$, pris conjointement, forment un groupe alkyle cyclique en $C_3$ à $C_6$ ; $R_5$ représente un atome d'hydrogène ou d'halogène ou un groupe alkyle en $C_1$ à $C_6$ droit ou ramifié, et ses sels pharmaceutiquement acceptables, sous réserve que le composé consistant en 2-[2-(diméthylamino)-5-fluoropyrimidine-4-yl]-1,5,6,7-tétrahydro-4H-pyrrolo[3,2-c]-pyridine-4-one soit exclu.

**6.** Composé de formule (I) suivant la revendication 5, dans lequel R représente un atome d'hydrogène, un groupe amino ou phénylamino ; $R_1$ et $R_2$ représentent l'un et l'autre un atome d'hydrogène ou bien, pris conjointement avec le noyau pyrimidine auquel ils sont liés, forment un groupe divalent -NH-CH=N- ; et $R_3$, $R'_3$, $R_4$, $R'_4$ et $R_5$ répondent aux définitions précitées.

**7.** Composé de formule (I) suivant la revendication 5, dans lequel $R_3$ et $R'_3$ représentent l'un et l'autre un atome d'hydrogène ou bien l'un d'entre eux représente un groupe phényle et celui restant représente un atome d'hydrogène ; et R, $R_1$, $R_2$, $R_4$, $R'_4$ et $R_5$ répondent aux définitions précitées.

**8.** Composé de formule (I) suivant la revendication 5, dans lequel $R_4$ et $R'_4$ représentent l'un et l'autre un atome d'hydrogène ou l'un et l'autre un groupe méthyle ; et R, $R_1$, $R_2$, $R_3$, $R'_3$, $R_4$, $R'_4$ et $R_5$ répondent aux définitions précitées.

**9.** Composé de formule (I) suivant la revendication 5, dans lequel $R_5$ représente un atome d'hydrogène et R, $R_1$, $R_2$, $R_3$, $R'_3$, $R_4$ et $R'_4$ répondent aux définitions précitées.

**10.** Composé de formule (I) suivant la revendication 5, facultativement sous forme d'un de ses sels pharmaceutiquement acceptables, choisi dans le groupe consistant en :

chlorhydrate de 2- (2-aminopyrimidine-4-yl) -1,5,6,7-tétrahydro-4H-pyrrolo[3,2-c]pyridine-4-one ;
chlorhydrate de 2-(2-aminopyrimidine-4-yl)-7-phényl-1,5,6,7-tétrahydro-4H-pyrrolo[3,2-c]pyridine-4-one ;
chlorhydrate de 2-(2-phénylaminopyrimidine-4-yl)-1,5,6,7-tétrahydro-4H-pyrrolo[3,2-c]pyridine-4-one ;
chlorhydrate de 2-(9H-purine-6-yl)-1,5,6,7-tétrahydro-4H-pyrrolo[3,2-c]pyridine-4-one ;
chlorhydrate de 2-pyrimidine-4-yl-1,5,6,7-tétrahydro-4H-pyrrolo[3,2-c]pyridine-4-one ;
chlorhydrate de 7-phényl-2-(9H-purine-6-yl)-1,5,6,7-tétrahydro-4H-pyrrolo[3,2-c]pyridine-4-one ;
chlorhydrate de 2-(2-aminopyrimidine-4-yl)-6,6-diméthyl-1,5,6,7-tétrahydro-4H-pyrrolo[3,2-c]pyridine-4-one ;
chlorhydrate de 7-phényl-2-pyrimidine-4-yl)-1,5,6,7-tétrahydro-4H-pyrrolo[3,2-c]pyridine-4-one ;
chlorhydrate de 2-(2-anilinopyrimidine-4-yl)-7-phényl-1,5,6,7-tétrahydro-4H-pyrrolo[3,2-c]pyridine-4-one ;

2-(2-aminopyrimidine-4-yl)-6-isopropyl-1,5,6,7-tétrahydro-4H-pyrrolo[3,2-c]pyridine-4-one ;
2-(2-aminopyrimidine-4-yl)-7-méthyl-1,5,6,7-tétrahydro-4H-pyrrolo[3,2-c]pyridine-4-one ;
2-(2-aminopyrimidine-4-yl)-6-méthyl-1,5,6,7-tétrahydro-4H-pyrrolo[3,2-c]pyridine-4-one ;
2-(2-aminopyrimidine-4-yl)-7,7-diméthyl-1,5,6,7-tétrahydro-4H-pyrrolo[3,2-c]pyridine-4-one ;
2-(2-aminopyrimidine-4-yl)-6-isobutyl-1,5,6,7-tétrahydro-4H-pyrrolo[3,2-c]pyridine-4-one ;
4-(4-oxo-4,5,6,7-tétrahydro-1H-pyrrolo[3,2-c]pyridine-2-yl)pyrimidine-2-ylcarbamate d'éthyle ;
(7R ou 7S)-2-(2-aminopyrimidine-4-yl)-7-méthyl-1,5,6,7-tétrahydro-4H-pyrrolo[3,2-c]pyridine-4-one ;
(7R ou 7S)-2-(2-aminopyrimidine-4-yl)-7-méthyl-1,5,6,7-tétrahydro-4H-pyrrolo[3,2-c]pyridine-4-one ;
2-(2-aminopyrimidine-4-yl)-3-iodo-1,5,6,7-tétrahydro-4H-pyrrolo[3,2-c]pyridine-4-one ;
2-(2-aminopyrimidine-4-yl)-7,7-diéthyl-1,5,6,7-tétrahydro-4H-pyrrolo[3,2-c]pyridine-4-one ;
2-{2-[(2-furylméthyl)amino]pyrimidine-4-yl}-1,5,6,7-tétrahydro-4H-pyrrolo[3,2-c]pyridine-4-one ;
N- [4- (4-oxo-4, 5, 6, 7-tétrahydro-1H-pyrrolo[3,2-c]-pyridine-2-yl)-pyrimidine-2-yl]-benzamide ;
2-(2-aminopyrimidine-4-yl)-7-isopropyl-1,5,6,7-tétrahydro-4H-pyrrolo[3,2-c]pyridine-4-one ;
2-[2-(benzylamino)pyrimidine-4-yl] -1,5,6,7-tétrahydro-4H-pyrrolo[3,2-c]pyridine-4-one ;
2-[2-(propylamino)pyrimidine-4-yl]-1,5,6,7-tétrahydro-4H-pyrrolo[3,2-c]pyridine-4-one ;
2-[2-(isobutylamino)pyrimidine-4-yl]-1-1,5,6,7-tétrahydro-4H-pyrrolo[3,2-c]pyridine-4-one;
2-{2-[(cyclohexylméthyl)amino]pyrimidine-4-yl}-1,5,6,7-tétrahydro-4H-pyrrolo[3,2-c]pyridine-4-one ;
2-{2-[(2-furylméthyl)amino]pyrimidine-4-yl}-1,5,6,7-tétrahydro-4H-pyrrolo[3,2-c]pyridine-4-one ; et
trifluoracétate de N-[4-({[4-(4-oxo-4,5,6,7-tétrahydro-1H-pyrrolo[3,2-c]pyridine-2-yl)pyrimidine-2-yl]amino}méthyl)-phényl]acétamide.

**11.** Procédé pour la préparation des composés de formule (I) et de leurs sels pharmaceutiquement acceptables suivant la revendication 5, procédé qui comprend :

a) l'halogénation d'un composé de formule (II), de manière à obtenir un composé de formule (III)

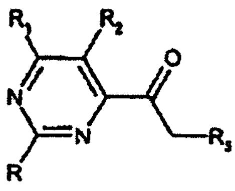

(II)

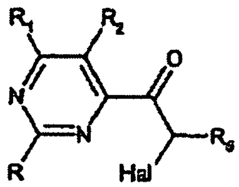

(III)

formules dans lesquelles R, $R_1$ et $R_2$ répondent aux définitions précitées, $R_5$ représente un atome d'hydrogène ou groupe alkyle en $C_1$ à $C_6$ droit ou ramifié et Hal représente un atome d'halogène convenable, de préférence un atome de brome ou de chlore ;
b) la réaction du composé de formule (III) avec un composé de formule (IV)

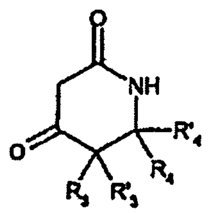

(IV)

dans laquelle $R_3$, $R'_3$, $R_4$ et $R'_4$ répondent aux définitions précitées, de manière à obtenir un composé de formule (I) et, facultativement, la conversion de ce composé en un autre composé de formule (I) et/ou en un de ses sels pharmaceutiquement acceptables.

**12.** Procédé suivant la revendication 11, dans lequel l'étape (a) est mise en oeuvre par bromation ou chloration du composé de formule (II).

**13.** Procédé suivant la revendication 12, dans lequel, dans le composé de formule (III), Hal représente un atome de brome ou de chlore.

**14.** Composition pharmaceutique comprenant une quantité thérapeutiquement efficace d'un composé de formule (I) ou d'un de ses sels pharmaceutiquement acceptables, tel que défini dans la revendication 5, sauf que le composé consistant en 2-[2-(diméthylamino)-5-fluoropyrimidine-4-yl]-1,5,6,7-tétra-hydro-4H-pyrrolo[3,2-c]pyridine-4-one n'est pas exclu, et au moins un excipient, support et/ou diluant pharmaceutiquement acceptable.

**15.** Composition pharmaceutique suivant la revendication 14, comprenant en outre un ou plusieurs agents chimiothérapeutiques.

**16.** Produit ou kit comprenant un composé de formule (I) ou un de ses sels pharmaceutiquement acceptables, tel que défini dans la revendication 5, sauf que le composé consistant en 2-[2-(diméthylamino)-5-fluoropyrimidine-4-yl]-1,5,6,7-tétrahydro-4H-pyrrolo[3,2-c]pyridine-4-one n'est pas exclu, ou une composition pharmaceutique le contenant telle que définie dans la revendication 14, et un ou plusieurs agents chimiothérapeutiques, sous forme d'une préparation combinée pour une utilisation simultanée, séparée ou séquentielle en thérapie anticancéreuse.

**17.** Composé de formule (I) ou un de ses sels pharmaceutiquement acceptables suivant la revendication 5, sauf que le composé consistant en 2-[2-(diméthylamino)-5-fluoropyrimidine-4-yl]-1,5,6,7-tétrahydro-4H-pyrrolo[3,2-c]-pyridine-4-one n'est pas exclu, destiné à être utilisé comme médicament.

**18.** Utilisation d'un composé de formule (I) ou d'un de ses sels pharmaceutiquement acceptables suivant la revendication 5, sauf que le composé consistant en 2-[2-(diméthylamino)-5-fluoropyrimidine-4-yl]-1,5,6,7-tétrahydro-4H-pyrrolo [3,2-c]pyridine-4-one n'est pas exclu, dans la production d'un médicament ayant une activité antitumorale.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 03027114 A
- WO 0212242 A
- WO 0069846 A
- WO 0198299 A
- WO 03014090 A
- WO 03028720 A
- WO 04058762 A
- US 60434962 B


### Non-patent literature cited in the description


- *Current Opinion in Chemical Biology,* 1999, vol. 3, 459-465
- **MONTAGNOLI A. et al.** *EMBO Journal,* 2002, vol. 21 (12), 3171-3181
- *J. Het. Chem.,* 1985, vol. 22 (6), 1723-6
- *J. Med. Chem.,* 1992, vol. 35, 3288
- *Tetrahedron,* 1997, vol. 53 (6), 2291-2302